# EUROPEAN PATENT SPECIFICATION

(11) **EP 3 760 170 B1**
(45) Date of publication and mention of the grant of the patent: **19.06.2024**
(21) Application number: 20465535.1
(22) Date of filing: 12.06.2020
(51) Int. Cl.: A61F 5/02, A61B 5/11, A61B 5/00, G08B 21/24, A61F 5/055, A63B 23/02, A63B 24/00

(54) **DEVICE FOR REAL-TIME MONITORING AND ACTIVE POSTURAL AUTOCORRECTION**
VORRICHTUNG ZUR ECHTZEIT-ÜBERWACHUNG UND AKTIVEN SELBSTKORREKTUR DER KÖRPERHALTUNG
DISPOSITIF DE SURVEILLANCE EN TEMPS RÉEL ET D'AUTOCORRECTION POSTURALE ACTIVE

(30) Priority: 01.07.2019 RO 201900398
(43) Date of publication of application: 06.01.2021
(73) Proprietor: Institutul National de Cercetare -Dezvoltare Pentru Mecatronica Si Tehnica Masurarii- I.N.C.D.M.T.M. Bucuresti, 021631 Bucharest (RO)
(72) Inventor: BADEA, Cristian Radu, 021181 Bucharest (RO)
(74) Representative: Fierascu, Cosmina-Catrinel

(56) References cited:
- CN-A- 109 009 600
- US-A- 3 608 541
- US-A- 4 007 733
- US-A- 4 750 480
- US-A1- 2009 062 706

## Description

The invention relates to a device used by a human user for maintaining a posture as correct as possible considering the physiological curvatures of the spine, as well as the shoulders positions during the bending / straightening movements of his torso and/or while performing weight lifting actions, during physical training, physiotherapy sessions, or while performing the movements required in various lucrative chores, as well as during normal household chores and walking. It is also used to perform active postural autocorrections, by the user, from the point of view of the physiological curvatures of the spine, as well as from the point of view of the shoulders position. The device performs a real-time monitorisation of the spine posture and the user's shoulders positions and signals any changes in relation to a previously established standard posture.

Several devices for postural monitoring and / or correction are known:
- CN109171750 (A) - presents a device that monitors the position consisting of a primary detection unit and / or a secondary detection unit, a processing unit and a display / warning reaction unit that are electrically connected to the processing unit. The detection unit includes a body that is attachable to the user's cervical area and a series of capacitive deformation sensors, arranged on the respective body. The secondary detection unit includes the following: a body covering the user's shoulder and a plurality of deformation sensors, resistive type, placed on the respective body. The processing unit is configured so that it generates prompt postural information, in accordance with the first information block concerning the deformations detected by the capacitive deformation sensors and / or in accordance with the second information block regarding the deformations detected by the durable deformation sensors. The display / warning reaction unit has the role of presenting / displaying the postural information captured by the previously mentioned sensors. Therefore, in accordance with the provided information, the user is able to carry out appropriate chores or relax / rest, thus avoiding the fatigue of the cervical spine and / or shoulders while maintaining a good health of the body;
- CN109147285 (A) - describes a portable, intelligent system for monitoring the posture during sitting (sitting in a chair posture), consisting of a module for monitoring the posture during sitting and a data processing module. The sitting posture monitoring module contains at least one flexible bending sensor, mounted with the active surface on the user's spine, in order to provide information about the changes experienced by the physiological curve of the monitored portion of the spine. The role of the data processing module is to decide whether the detected position is correct, otherwise it emits warning signals. The main advantage of the system is that it eliminates the physical discomfort created by mechanical postural correction systems. Another advantage of the invention is the real-time monitoring of the curves of the user's spine; the information delivered to the user is quickly processed and the obtained measurements have a quantitative nature;
- CN109009600 (A) - refers to an intelligent device for physiotherapy based on the deformation of sensitive elements, belonging to the category of equipment for health maintenance. According to the invention, the device contains a bone plate, a fastening belt, deformation sensitive elements, a microprocessor, an alarm system and a vibrating instrument for acupuncture or permanent vibrating magnets. The bone plate has the shape of the letter "I"; it is fixed and forms a whole unit with the fastening belt by means of a locking system. The deformation sensitive elements are placed at the two ends of an upper transverse plate respectively in the middle of a lower transverse plate, or on the lower area of a neutral plate. Once the user's spine deforms in relation to the normal physiological curves, thus deforming the bone plate, fixed on the user's body, the deformation sensitive elements release electrical signals that are processed by the microprocessor and transformed into alarm signals, in the form of photoelectric, vibrational and radio frequency signals. Acupuncture tools that use vibrations or permanent vibrating magnets are placed at both ends of the upper transverse plate and of the lower transverse plate of the bone plate. This invention is able to perform a direct and intelligent postural correction and it can also be used for massage and physiotherapy;
- US2019038006 (A1) - this invention refers to a postural support (1), comprising a wedge-shaped body (3.52x, 131) manufactured of an elastic, deformable material, with the possibility of a temporary fastening (detachable / separable) placed on a backpack (151). The invention further refers to a backpack (151) combined with the postural support (1), the latter being easily attachable or detachable on or off the backpack (151). Therefore, once the postural support (1) is attached to the backpack (151), the postural support (1) can alleviate the pressure exerted by the weight of the backpack (151) on the spine. Once the postural support (1) is detached from the backpack (151), it can be used as a postural support in the "sitting" position. It also relieves the pressure on the spine, caused by sitting on a flat surface. The postural support (1) is relatively inexpensive and it can be produced at significantly lower costs than similar supports;
- RO131307 (A0) - this invention refers to a method and to a wearable equipment with the purpose of an active correction of spinal deformities. The equipment according to the invention comprises several sensor blocks, a central processing and control module, an energy module and an interaction with the user module, in which the sensors are grouped in three categories: the flexible sensors that are used to determined the patient's posture and are installed on corrective clothing, very tight to the body, however allowing normal body movements; 3D sensors are also integrated in the corrective clothing. They are designed to determine the spatial position of the spine in relation to the vertical; the biosensors for ECG and for measuring the body temperature, the respiration rate and amplitude, in which the central processing and control module analyzes the patient's position; the module that interacts with the user and tries to restore the body position by electrostimulating the muscle groups. It also tries to obtain a voluntary action from the user, by signaling with vibration or sounds;
- US2005/0209645 (A1) - this invention is a medical device used to collect information on posture for the purpose of evaluating the therapy applied to a patient. The device collects information on posture based on the signals received from a series of sensors, such as the accelerometer, gyroscope or magnetometer, positioned orthogonally with respect to each other and aligned with the symmetry axis of the patient;
- US6095991 (A) - this invention relates to devices and methods for monitoring and recording the position and degree of movement of the body of a human or an inferior animal. The main proposed embodiment of the invention comprises: a 3D position monitoring system (1) based on a mercury-based sensor, a programmable control unit (2) which receives signals from the mercury-based sensor and interprets these signals in order to determine the appropriate position and movements of the area in which the respective sensor is located on the user's body, a storage device (3), operable by the respective control unit, which stores time data related to the position of the user's body and movements and an interface device (4) that is capable to transfer the stored data to an external computer. This invention eliminates the requirement of ambulatory recording, according to which the monitors have to be connected to external data storage and processing devices with cables, which often limits the performance of the monitoring chores only at the level of clinical laboratories;

- US6514218 (B2) - this invention refers to a device that monitors the posture and measures the user's respiratory parameters. It comprises a postural detection unit, which is based on a rotary position sensor, having a housing, an insulating element and a mobile body, on which a conductive element is installed. The latter moves freely inside the housing, where the contact points are placed at equal intervals around the central axis of the housing, on perpendicular levels. The surfaces also contain conductive elements. Therefore, once the mobile body moves, it will short-circuit two adjacent contacts. By placing it on the user's body, it can provide information on any posture changes as well as modifications in the respiratory rhythm of the respective user;
- US5199940 (A) - this invention refers to a device used for postural training and correction of the user and it comprises a rigid bar, firmly fixed along the spine, by using elastic bands placed at the head, shoulders and the pelvis of the user. The training and correction consists of the effort made by the user to maintain their posture so that their spine remains aligned with the rigid bar;
- US20080195010 (A1) - this invention refers to an assembly of elastic, adjustable bands, designed as a vest, used for postural correction. It comprises an elastic band adjustable at the waist. The band is equipped at the back with an elastic strap. The elastic strap is equipped with two other elastic straps fixed at the top, with adjustable length. It can be fastened around the user's shoulders;
- WO2010014027 (A1) - this invention refers to a device used to signal the user's postural changes, in order to identify the need of a postural correction. It comprises a mercury-based tilting sensor, a wheel meant to adjust the sensitivity of the tilting sensor, a sound generator, a signal delay circuit, which delays the indication of postural change when the user is constantly moving and a battery. The device compliant with the invention, is placed on the user's body, at the torso or neck level, using a belt or a chain;
- WO2006059917 (A1) - this invention refers to a device used to warn the user about changes in their posture. It is designed as a small backpack, attached to the user's body using elastic straps with adjustable length. It consists of a housing containing an electronic module for sound emissions. The sound signals may consist of a song or just a vibration. It also contains a sliding contactor and two batteries. Once the user's posture changes, due to the stretching of the elastic straps, the active elements of the sliding contactor slide relative to each other, thus causing the contactor to switch from "off" to "on" and to trigger an audio signal to alert the user about the need to adjust their posture;
- US20150290020 (A1) - this invention refers to a device used for correction of the cervical area by stretching and comprises a belt, a central joint element, placed on the belt and designed to be worn on the back of a user's neck. It can be used as a support point for the extension of the cervical spine, two shoulder supports attached to the belt on both sides of the central joint element. They facilitate the external rotation of the shoulders by using the belt and a pair of straps fastened to the belt. At the free ends they have a loop that enables an easy operation for the user. The shoulder supports are positoned between the central joint element and the pair of straps;
- RO131307 (A0) - this invention refers to a method and a wearable equipment, for the active correction of spinal deformities and comprises several sensor blocks, a central processing and control module, an energy module and a module for the interaction with the user, where the sensors are grouped in three categories: flexible sensors, to determine the patient's posture, installed on a corrective clothing, very tight to the body, but allowing normal body movements, 3D sensors also integrated in the corrective clothing. They are designed to determine the spatial position of the spine in relation to the vertical; the biosensors for ECG and for measuring the body temperature, the respiration rate and amplitude, in which the central processing and control module analyzes the patient's position; the module that interacts with the user and tries to restore the body position by electrostimulating the muscle groups. It also tries to obtain a voluntary action from the user, by signaling with vibration or sounds.
- US3608541 (A), US2009/0062706 (A1), US4007733 (A) and US4750480 (A) refer to devices used for correction of poor postures including different types of detectors or to methods of reprogramming dysfunctional soft tissue.

Each of the inventions summarised above, has different disadvantages compared to the current invention. Some of the most important ones are listed below:
- considering the comfort, some of them impose considerable physical constraints and discomfort for the user during use;
- considering the operation, they are cumbersome and involve the presence of a third party in order to apply the necessary corrections;
- some of them do not allow the use by people with certain anatomical specificities.
- considering their construction, they are difficult to manufacture as they involve the use of specialized sensors (mercury based tilting sensors, bending sensors), or a large number of sensors and complex electronic systems for detecting posture changes. They all lead to a substantial increase of the cost price;
- some of them require display systems (monitors), which further increase the cost price;
- some of them are intended for monitoring only certain segments of the spine, and others require a successive repositioning of the user in order to monitor the whole spine;
- some of them are intended exclusively to monitor / correct the spine, or the position of the shoulders;
- only a small part of those presented above allow the placement / use under the user's clothes, and in the situation when this is possible, due to the rather extended contact with the user's skin, they produce local overheating and / or irritation of the respective area;
- those that contain straps / belts that are to be placed around the shoulders can cause considerable local discomfort, as they cross and come into contact with the user's armpit areas. It is known that these areas have an increased level of sensitivity / irritability;
- some of them are not wearable, or can hardly be used in household, lucrative chores, or during physical training;
- some of them restrict even the normal, correct movements of the user;
- some of them are intended exclusively for posture monitoring or posture correction;
- some of those described above, are based on elastic elements, such as elastic bands / belts, with a relatively short lifespan, subject to early aging due to temperature differences and chemical factors (such as rain or perspiration);
- some of them are addressed only to hospital environment;
- due to the use of complex electronic systems, some of them cannot be used by people with pacemaker implants, as there is a risk of interference between their signals and the pacemaker;
- the devices that use sensors, have a high energy consumption, because they require a continuous power supply during the use of the invention.

According to the textbooks on the subject, the correct anatomical position during walking and while performing movements such as bending/straightening of the torso, weight lifting, from the point of view of the upper body, is where the spine maintains its normal physiological curves with the shoulders "slightly" rotated backwards and lowered in a relaxed position in frontal plane (called the neutral position of the shoulders). Bending /straightening and lifting is performed by complying with the aforementioned conditions, only by flexing the torso at the level of the hip joints. Moreover, according to the textbooks on the subject, the postural correction can be achieved gradually, by internal and / or external means, as a reaction of adaptation of the human body to gradual postural changes, for example by forcing the respective person to achieve and maintain a certain degree of postural correction. Once the objective is fulfilled a new degree of correction is set and so on, until the maximum possible level of correction is reached, taking into account the anatomical particularities of that person.

The main purpose of the current invention is to provide the user with a device that allows him to fulfill as accurately as possible, the postural requirements that must be observed during the bending / straightening movements of the torso and weight lifting, required during the physical training / physiotherapy trainings or those imposed within the various lucrative chores, as well as during the usual household chores. The previously mentioned requirements are:
- maintaining in the sagittal plane the physiological curvatures of the spine, by the user;
- maintaining the collinearity of the vertebrae in the frontal plane, by the user;
- maintaining the correct position, considering the requirements of the biomechanics of the bending / straightening motions of the torso, as well as of the position of the shoulders. The correct position of the shoulders is reached when the shoulders are lowered and "pulled" backwards, namely the scapulo-humeral joints are in external rotation.

The secondary purpose of this invention is to allow the user to gradually make postural corrections that observe the anatomical needs and particularities of the user. Therefore in case of a user with certain deviations of the spine, for example kyphosis, lordosis, the user is able to gradually make greater or lesser corrections, over time, which lead to a satisfactory final result, taking into account the specific pathology of the user, without necessarily requiring physiotherapy exercises to correct spinal curves.

The tertiary purpose of this invention is to allow the user to achieve and maintain a correct posture of the spine during walking.

The problems solved by the invention are the following:
- the accuracy of the bending / straightening movements of the torso and weight lifting, performed by the user, required during the physical trainings, or those imposed during the various lucrative chores, as well as during the usual household chores;
- making small postural corrections of the spine and shoulders of users with various changes in normal physiological curvatures of the spine, which combined can lead to a major correction of their posture;
- maintaining a correct posture of the user's spine while walking.

The device according to the invention, related to the proposed constructive version, comprises a central electronic unit, fastened on an adjustable abdominal belt, with two electrodes, connected to its outside, a flexible and inextensible thoracic-lumbar wire, which bifurcates to the left and right side, resulting in a left shoulder wire and a right shoulder wire, having their ends opposing the bifurcation point, fixed on a left shoulder strap-type support, respectively on a right shoulder strap-type support and a fastening support. The thoracic-lumbar wire continues on the upper part of the bifurcation point, with a cervical-thoracic wire, whose terminal end is fixed on a cervical strap-type support. The three strap-type supports (right shoulder strap-type support, left shoulder strap-type support and cervical strap-type support) are made identically from a constructive point of view. For example, the left shoulder strap-type support comprises a strap produced of a flexible material (preferably rubber with textile insertion for ensuring tearing strength), on whose lower surface is fixed a reusable double-sided adhesive tape (with biocompatible adhesive), flexible and compressible, an adjusting body in the form of a spool, rotatably fastened on an anti-rotation body embedded in the strap, on its upper surface. The left shoulder wire is wound on the adjusting body of the left shoulder strap-type support, the right shoulder wire is wound on the adjusting body of the right shoulder strap-type support. The cervical-thoracic wire is wound on the adjusting body of the cervical strap-type support.

The central electronic unit comprises a case inside which there is an electronic module, that is connected to a linear potentiometer, a drive body fixed on the potentiometer's rod, slides through the grooves of a guide body, that is fixed on the electronic module's circuit board, a tension spring, has it's lower end anchored on a rotating hook, placed on the guide body and its upper end fixed on the lower end of the drive body, a bush is crimped on the lower end of the thoracic-lumbar wire, which enters through a circular opening in the upper wall of the case, is used to hold the thoracic-lumbar wire fixed on the upper end of the drive body, a retaining knob is screwed into the left side wall of the case; its shaft penetrates through a recess in the left side wall of the guide body, thus allowing the free end of the retaining knob's shaft to reach in the vicinity of the drive body, an ON / SET LED, a speaker, a setting button, a ON / OFF button, a low level indicator LED, a medium level indicator LED, a high level indicator LED, a lid, a "female" connector, a "male" connector that contains the ends of the two electrode cables, an elastic clip, placed on the housing and fixed with a screw.

The fastening support consists of two rigid arms, left and right, respectively, a locking knob, screwed on the connecting ends of the two arms mentioned above, a flexible body, that has its ends rigidly fixed on the outer ends of the two rigid arms, two pairs of magnets, one pair on each of the outer ends of the rigid arms and another pair of magnets embedded on the upper face of the flexible body. The first two pairs of magnets are embedded on the lower faces of the two rigid arms.

The device according to the invention has the following advantages:
- it allows real-time monitoring of postural changes in the user's spine and shoulders;
- it allows the active and fast autocorrection of the user's torso posture, in regard with his spine and shoulders, in relation to a predetermined posture, that can be the physiological posture, indicated by the textbooks on the subject, or the most correct posture that can be attained considering the anatomical particularities of the user;
- it can be used to maintain the most correct posture of the spine and shoulders, by a human user, during the bending / straightening motions of its torso, during physical training / physiotherapy or various lucrative chores, as well as during ordinary household chores;
- it can be used to achieve, gradually postural corrections, namely, in the case of a user who has certain deviations of the spine, such as kyphosis, lordosis or scoliosis, this allowing the user to make in time, small corrections that together lead to a satisfactory final result taking into account the specifics of the user's pathology, without necessarily requiring the performance of physiotherapeutic exercises to correct the curves of the spine, which are often very time consuming chores and demanding in terms of the physical and mental effort it requires;
- it can be used in combination with physiotherapy exercises, thus reducing the necessary recovery time of the user;
- it is portable and occupies a relatively small space; it can be worn under clothes, due to the reduced contact between the forming parts of the device and the user's skin, the risk of local overheating or irritation is relatively low;
- it is easy to use and maintain;
- it is adaptable depending on the biometric measures (height and distance between the shoulders) of the user and according to the pathological specificities of their spine;
- the device can also be used by people with certain anatomical features, such as: clear differences between the lengths of the two lower limbs and / or between the upper limbs, various deviations of the spine, an extraordinary bone / muscle structure;
- the device can send warnings concerning the change of the user's posture. The warnings may consist of acoustic signals or electrical impulses with a low duration and intensity ("mini-electric shock"), applied in high muscle mass areas (for example buttocks or shoulders);
- the device allows very fast autocorrections, due to the warning mode that uses electrical impulses of low duration and intensity ("mini-electric shock");
- the device allows adjusting the sensitivity level, depending on the needs / preferences of the user;
- the device allows the warning of the user not only with regard to a change of the posture of the lumbar-thoracic spine, but also of their cervical spine;
- the device allows the warning of the user about the change of the posture of the spine and also of the shoulders;
- the device allows the warning of the user about the simultaneous change of shoulder positions and also about the change of position of each shoulder;
- the device allows alerting the user about the postural changes of the spine both in the sagittal and frontal planes;
- the device allows the user to omit the control of the changes appeared in the area of the cervical curve, or the control of the changes occured in the shoulders area;
- the device can be worn by using the included belt or by fastening it with the aid of an elastic clip, on the belt of the pants / skirt, or on the waistband of the user's pants / skirt, or on the weight lifting belt, used for protection during strength training;
- the device can be used in the same way, regardless of the user's sex, age and race;
- the device can also be used by people with pacemaker implants because no emission of a radio frequency signal occurs and there is no electrical contact with the skin, so that there is no possibility of interference with the signals compared to the aforementioned devices;
- the device can be used daily, regardless of the environment in which the user operates, due to its small size and weight. It also does not restrict the natural movements of the user's body, as long as they are performed correctly from a biomechanical point of view;
- the device allows the user to opt for postural autocorrection only considering their spine position or their shoulders position, or both anatomical elements.

An embodiment of the invention is presented below, in connection with fig.1, fig.2, fig.3, fig.4, fig.5, fig.6, fig.7, fig.8, fig.9, fig.10, fig.11, fig.12, fig.13, fig.14, fig.14 and fig.15 which illustrate the following:
- fig.1 - a perspective view of the device, according to the invention;
- fig.2 - a perspective view of the rear side of the central electronic unit of the device, according to the invention;
- fig.3 - the front view of the central electronic unit of the device, according to the invention;
- fig.4 - a sectional view in a horizontal plane, at the level of the axis of rotation of the locking knob of the central electronic unit of the device, according to the invention;
- fig.5 - perspective view of the support of the left shoulder strap-type, sectional view in a vertical plane at the level of the adjusting body of the left shoulder strap-type support and a detailed view of the anti-rotation body of the support of the left shoulder strap-type support of the device, according to the invention;
- fig.7 - a sectional view made in a vertical plane, at the level of the adjusting body of the left shoulder strap-type support of the device, according to the invention;
- fig.8 - detailed view at the level of the anti-rotation body of the left shoulder strap-type support of the device, according to the invention;

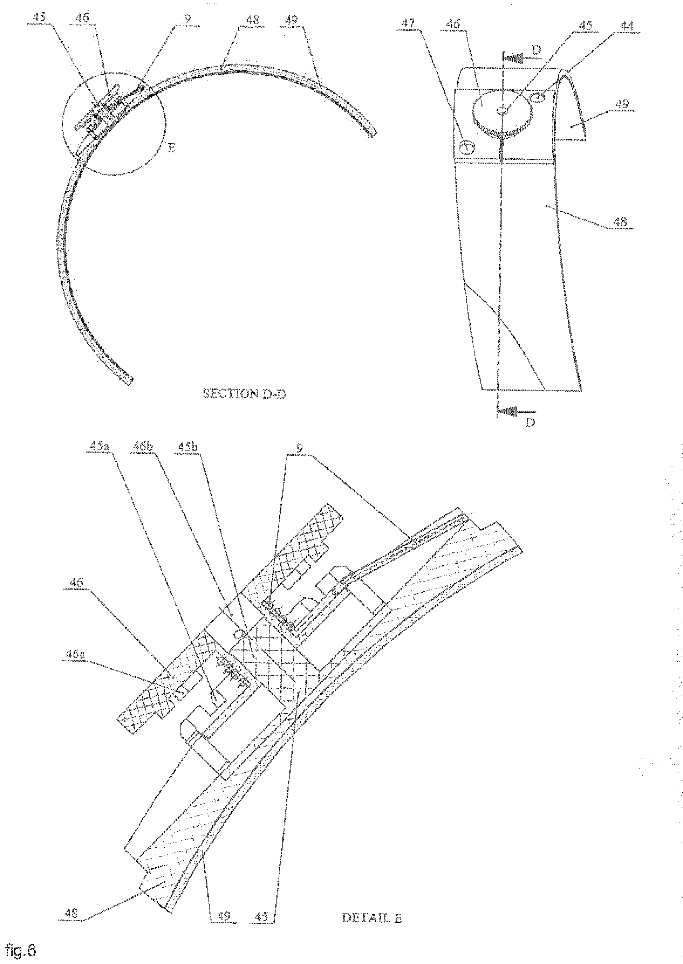

- fig.6 - detailed view at the level of the anti-rotation body of the left shoulder strap-type support of the device, according to the invention, with the adjusting body displaced in the position required for tensioning the left shoulder wire;

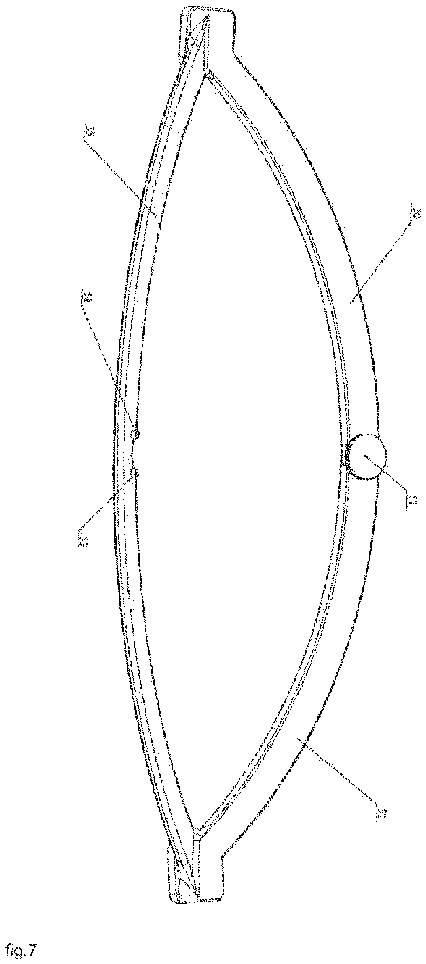

- fig.7 - perspective view of the upper side of the fastening support of the device, according to the invention;

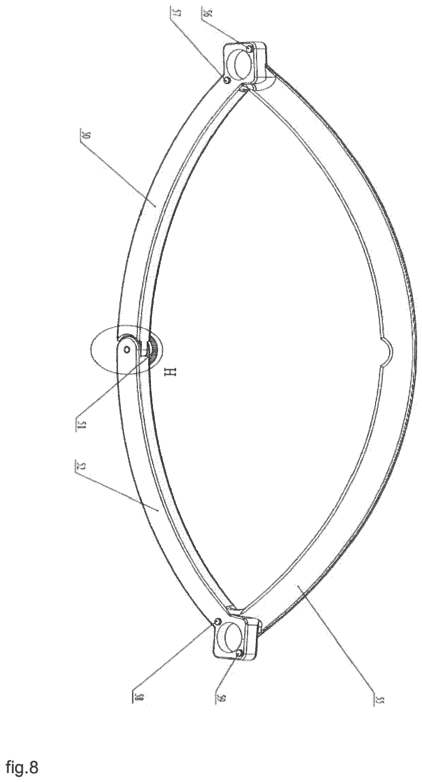

- fig.8 - perspective view of the lower side of the fastening support of the device, according to the invention;

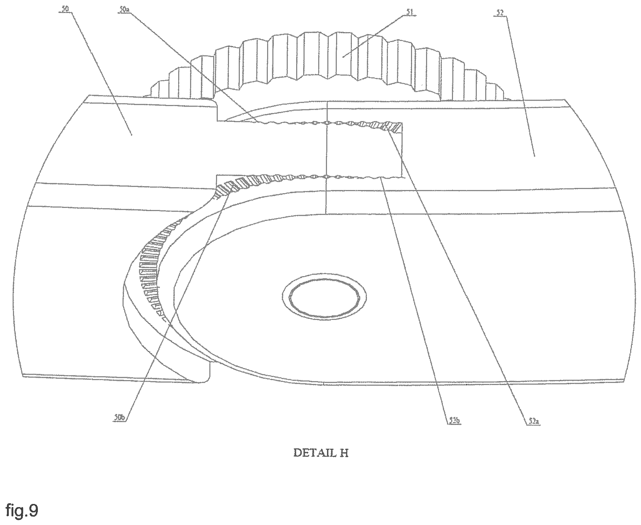

- fig.9 - detailed view at the level of the locking knob of the fastening support of the device, according to the invention, with the highlighting of the radial grooves applied on the surfaces in contact with each other, of the two rigid arms contained within the fastening;

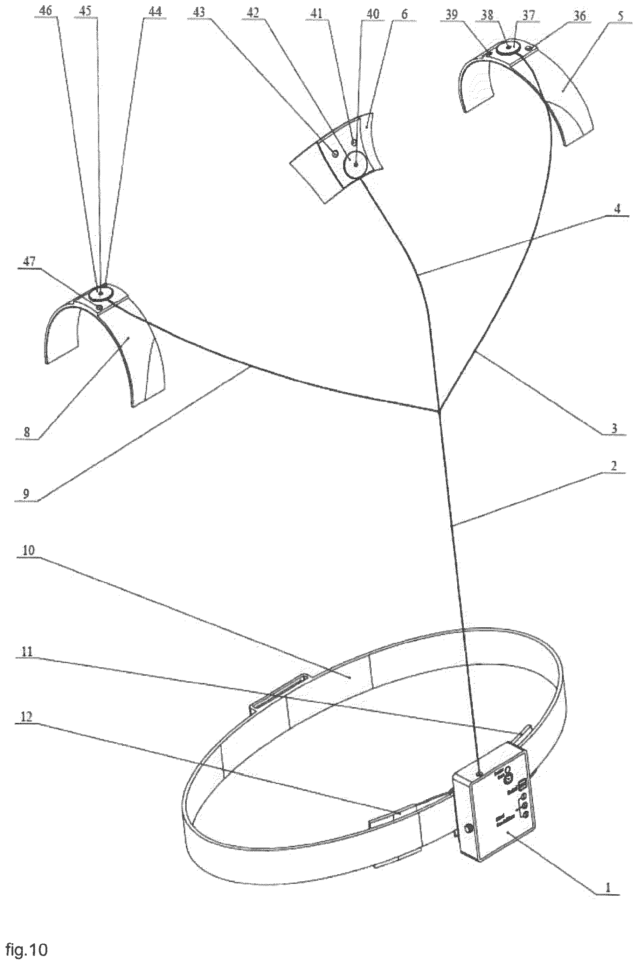

- fig.10 - perspective view of the device, according to the invention, having the fastening support removed;

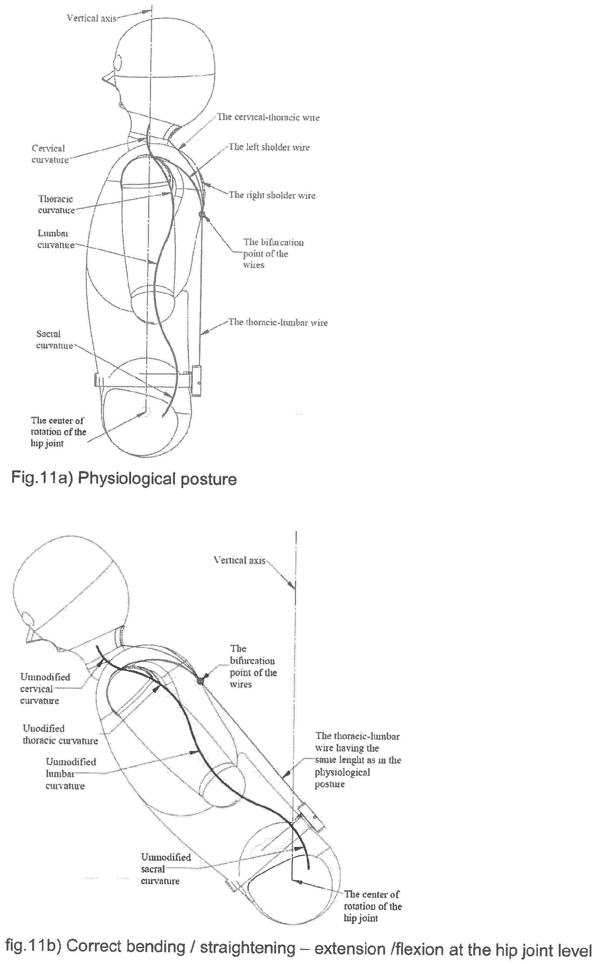

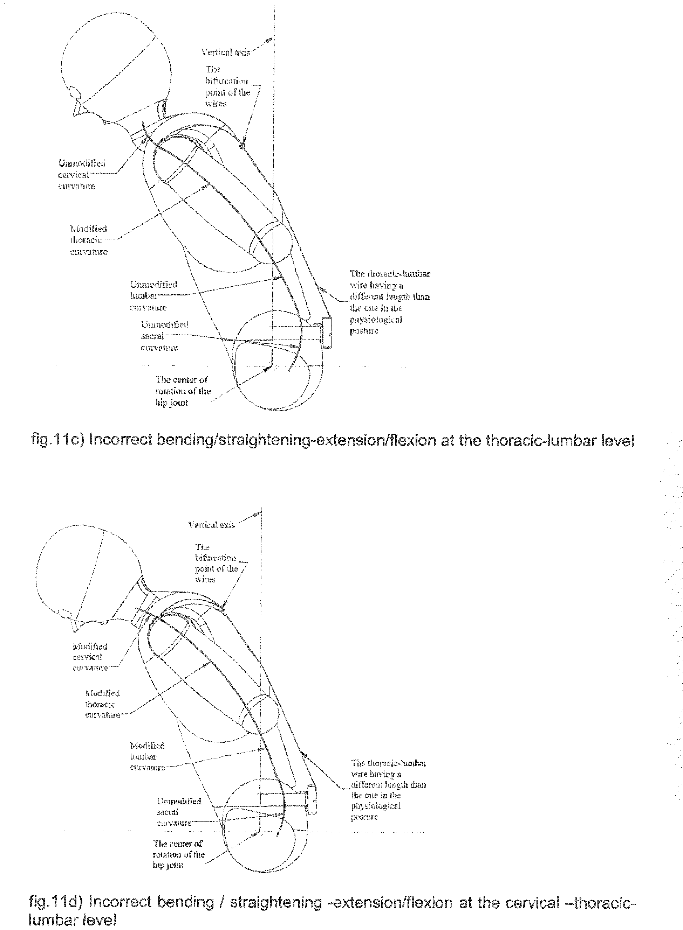

- fig.11 - side view of the device, according to the invention, with it being mounted on a user, highlighting the physiological curves of the spine before performing a bending / straightening (posture imposed in the calibration stage of the device - check figure 11.a), during a bending / straightening movement that does not cause abnormal changes in the curvatures of the user's spine the bending / straightening movement is performed via a hips joint flexion, maintaining the physiological curvatures of the spine, to underline the maintaining of the equality between the length of the thoracic-lumbar wire in the correct orthostatic position, during the calibration of the device and the length in the bent position - check figure 11.b), during a bending / straightening movement that causes an abnormal change of the lumbar and thoracic curves (the bending / straightening movement performed via a flexing at the lower and upper abdominal level, to underline the change in the length of the thoracic-lumbar wire after the bending movement achived by lumbar-thoracic flexion / extension, in relation to the length during the calibration of the device - check figure 11.c), as well as during the execution of a bending / straightening movement that causes abnormal modification of the lumbar, thoracic and cervical curvatures bending / straightening movement being performed by flexion at the lower and upper abdominal level, as well as at the neck level, to underline the change in the length of the thoracic-lumbar wire after the bending movement by lumbar-thoracic-cervical flexion / extension , in relation to the length during the calibration of the device - check figure 11.d);

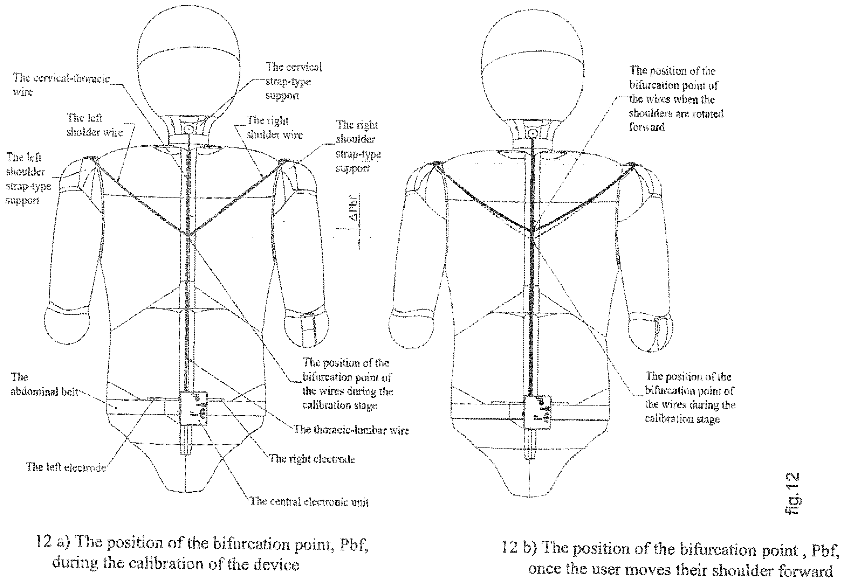

- fig.12: Fig 12.a) - front view of the device, according to the invention, mounted on the user's torso highlighting the position of the bifurcation point, Pbf, during the calibration of the device, and Fig 12.b) , highlighting the displacement of the bifurcation point of the device's wires, ΔPbf, after the user has rotated his shoulders forward;

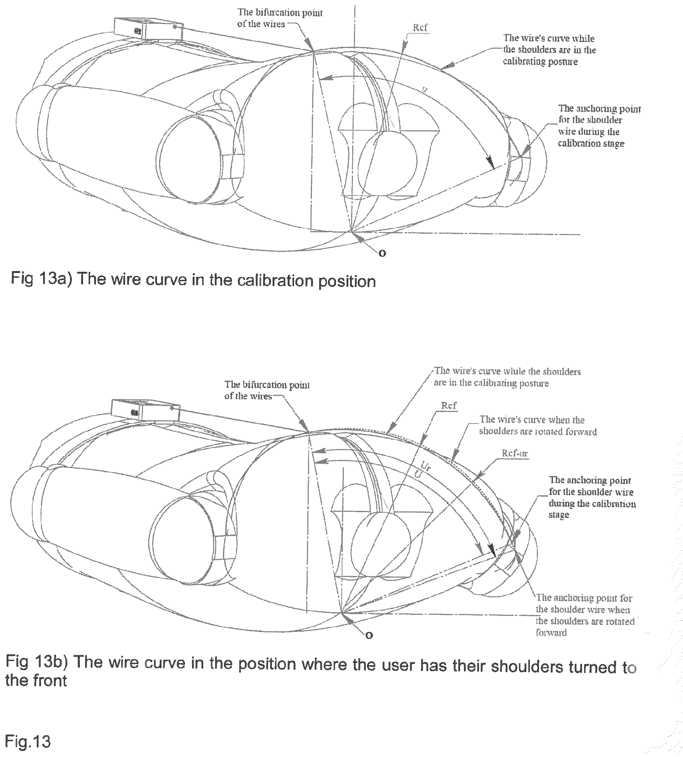

- fig.13: Fig 13.a) sectional view with a cutting plane containing the bifurcation point of the wires, the end and the middle point of the left shoulder wire of the device mounted on a user, according to the invention, and Fig 13.b) highlighting the curve of the left shoulder wire during the calibration of the device;

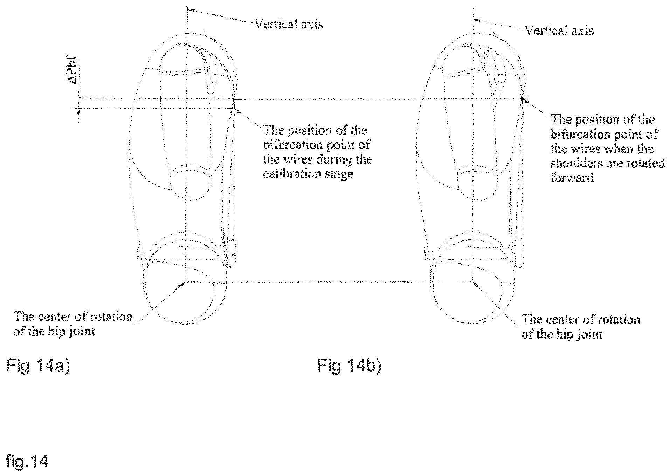

- Fig. 14: Fig 14.a) side view of the device, according to the invention, while being worn by a user, highlighting the position of the bifurcation point of the wires in the calibration stage, and Fig 14.b) side view of the device, while being worn by a user, highlighting the displacement of the bifurcation point of the wires, ΔPbf, after the user rotates his shoulders forward;

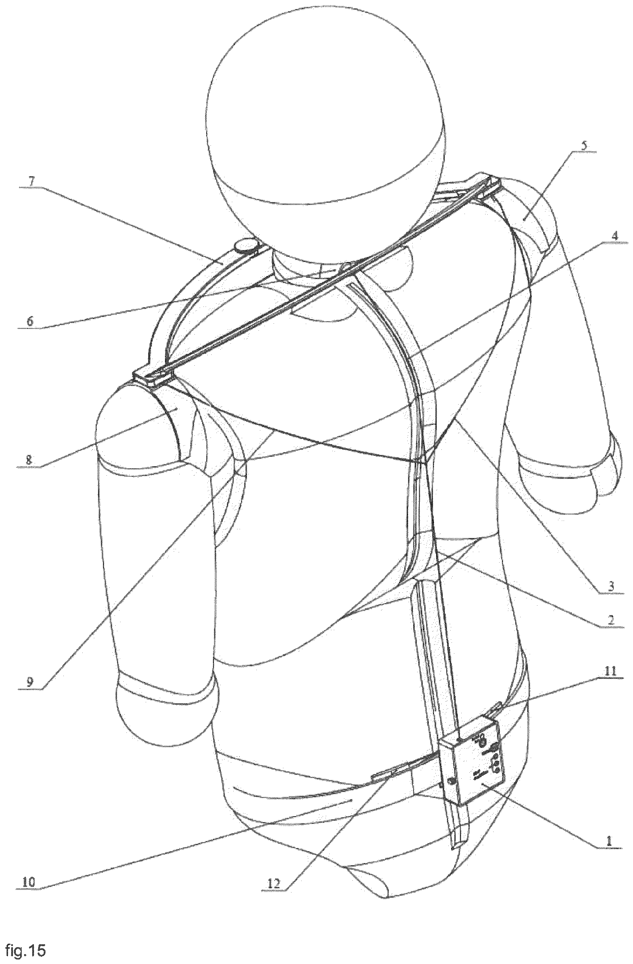

- Fig. 15 perspective view of the device, according to the invention, that highlights its placement on the user's body.

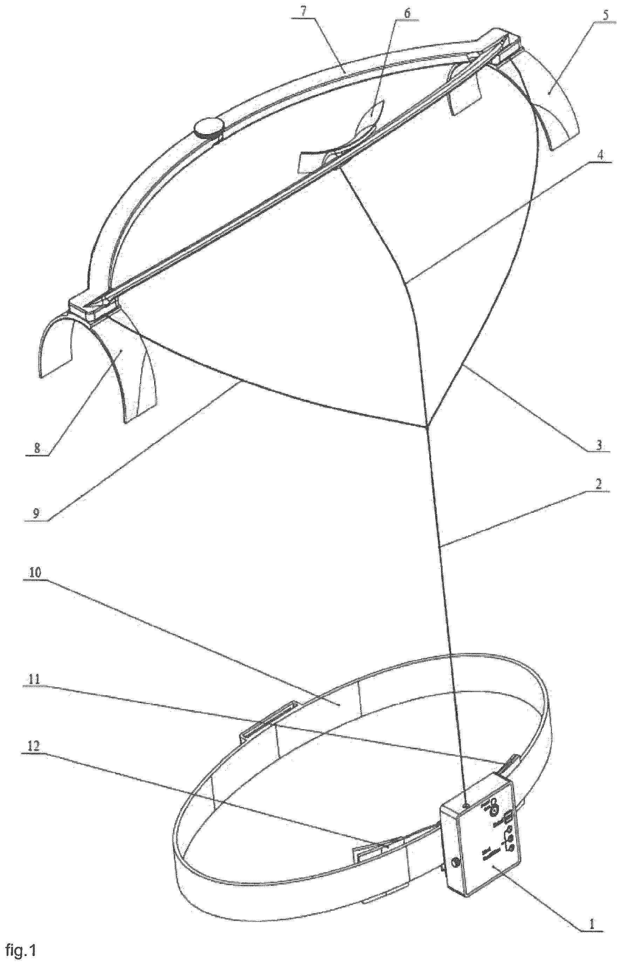

The device according to the invention, in relation with the suggested constructive version comprises a central electronic unit 1, fastened on an adjustable abdominal belt 10, with two outside connected electrodes, the right 11 and respectively left 12, a flexible and inextensible thoracic-lumbar wire 2, which bifurcates sideways left and right, resulting in a left shoulder wire 9 and a right shoulder wire 3, having its ends opposing the bifurcation point, fastened on a left shoulder strap-type support 8 and respectively on a right shoulder strap-type support 5, the thoracic-lumbar wire 2 is continuing to the upper side, from the bifurcation point, with a cervical-thoracic wire 4, whose end is fastened on a cervical strap-type support 6 and a fastening support 7 (check fig.1).

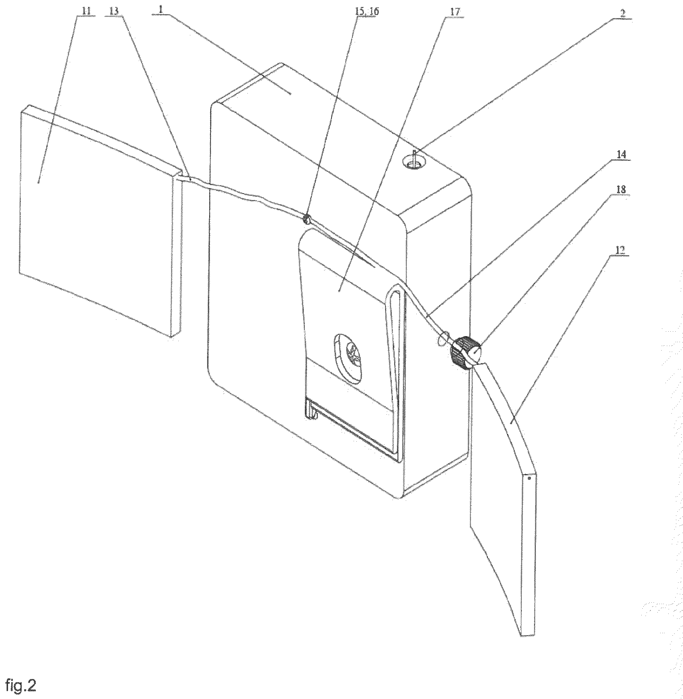

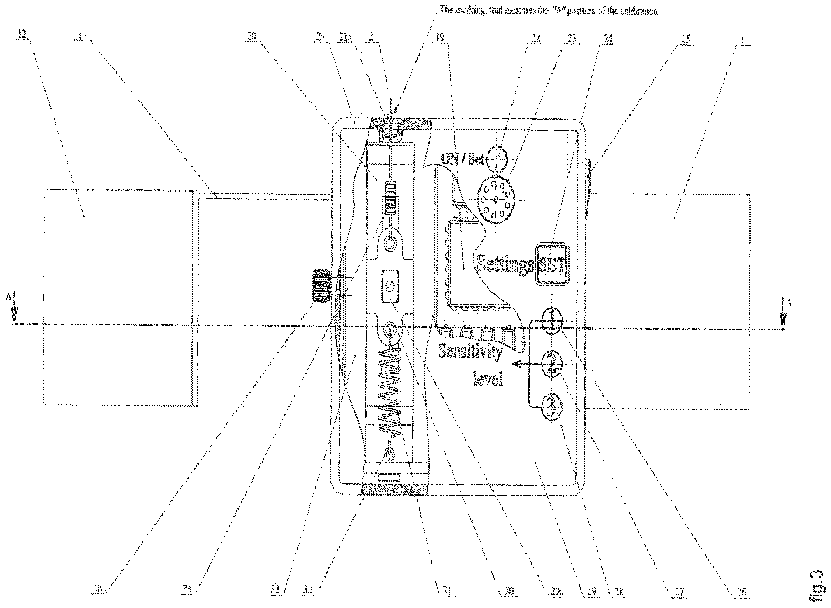

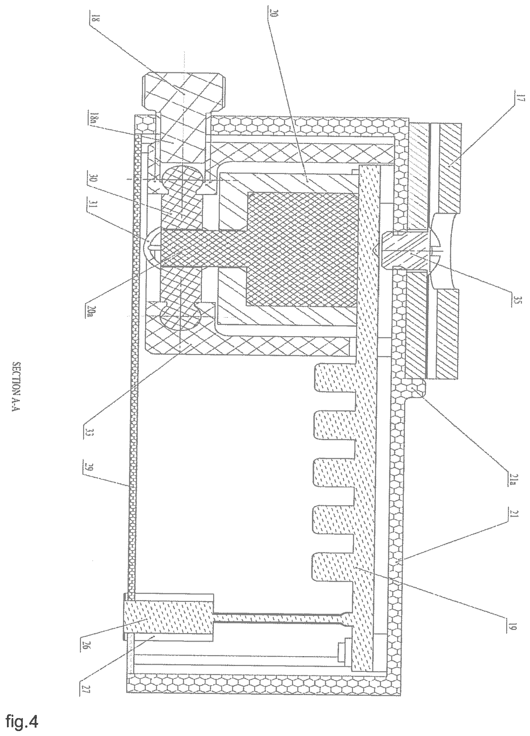

The central electronic unit comprises a case 21, inside which there is an electronic module 19, that is connected to a linear potentiometer 20, a drive body 30, fixed on the potentiometer's 20 rod, slides through the grooves of a guide body 33, that is fixed on the electronic module's 19 circuit board, a tension spring 31 has its lower end anchored on a rotating hook 32, placed on the guide body 33 and its upper end fixed on the lower end of the drive body 30, a bushing 34 crimped on the lower end of the thoracic-lumbar wire 2, which enters through a circular opening in the upper wall of the case 21, is used to hold the thoracic-lumbar wire fixed on the upper end of the drive body 30, a retaining knob 18 is screwed into the left side wall of the case; its shaft penetrates through a recess in the left side wall of the guide body 33, thus allowing the free end of the retaining knob's shaft to reach in the vicinity of the drive body 30 (check fig.4), an ON / SET LED 22, a speaker 23, a setting button 24, a ON / OFF button 25, a low level indicator LED 26, a medium level indicator LED 27, a high level indicator LED 28, a lid 29 (check fig.3), a "female" connector 16, a "male" connector 15, that contains the ends of the two cables, right 13 and respectively left 14, of the electrodes right 11 and respectively left 12 and an elastic clip 17, placed on the case 21 and fixed with a screw (check fig.2, fig.3 and fig.4).

The purpose of the drive body 30 is to move the rod 20a, of the linear potentiometer 20, under the action of movements produced by the tension of the thoracic-lumbar wire 2, thus modifying the electrical voltage at the output terminals of the respective potentiometer.

The purpose of the compression spring 31 is to maintain / return the rod 20a, of the linear potentiometer 20, in the calibration position of the device, according to the invention.

The purpose of the retaining knob 18 is to block the movement of the drive body 30 and so of the rod 20a, of the linear potentiometer 20, in order to allow the calibration of the device, according to the invention.

The purpose of the bushing 34 is to block the opening of the loop that attaches the thoracic-lumbar wire 2 to the drive body 30.

The ON / SET LED 22 has the following functions:
- to confirm the start /stop of the device in question. The action is performed by means of the ON / OFF button 25;
- to confirm the entry of the current device in the state in which the sensitivity level of the device can be set. This is achieved by emitting intermittent light signals;
- to confirm the completion of setting the sensitivity level of the device in question. This is achieved by emitting a continuous light signal;
- to identify, by changing the wavelength of the emitted beam and by switching to an intermittent light signal, whether the sensitivity level set by the user is exceeded.

The purpose of the speaker 23 is to confirm, by emitting a continuous and short-term sound signal, the start of the device in question, as well as to warn the user, by means of an intermittent sound signal, if the sensitivity level set by the user was exceeded. The latter type of signal will stop once the device returns below the sensitivity level set by the user.

The purpose of the setting button 24 is to allow the user to set the desired sensitivity level. Switching from one sensitivity level to another is possible by successively pressing this button. Once the desired level is reached, the confirmation consists of emitting continuous light signals, by one of the low level indicator LED 26, medium level indicator LED 27, or high level indicator LED 28, as follows:
- the illumination achieved by the low level indicator LED 26, confirms the setting of a low sensitivity level for the respective device. Considering the purpose of the device, only a substantial change of the user's posture will trigger an audible warning, emitted by the speaker 23 and the emission of an intermittent light signal by the low level indicator LED 26;
- the illumination achieved by the medium level indicator LED 27, confirms the setting of an average sensitivity level for the respective device. Considering the purpose of the device, only an average change of the user's position will trigger an audible signal, emitted by the speaker 23 and the emission of an intermittent light signal by the medium level indicator LED 27;
- the illumination achieved by the high level indicator LED 28, confirms the setting of a high sensitivity level of the respective device. Considering the purpose of the device, the slightest change in the user's posture will trigger an audible signal, emitted by the speaker 23 and the emission of an intermittent light signal by the high level indicator LED 28.

The setting button 24 is placed so that its upper face is below the level of the upper face of the lid 29, so it does not allow an accidental pressing during the use of the device, according to the invention.

Each of the three levels of sensitivity, mentioned above, corresponds to a certain length of the stroke made by the rod 20a, of the linear potentiometer 20, determined by the traction exerted on the thoracic-lumbar wire 2, by any of the wires cervical - thoracic 4, left shoulder 9 and / or right shoulder 3, once the user changes their posture by comparison to the posture upon the calibration of the respective device.

The purpose of the ON / OFF button 25 is to trigger the power supply of the central electronic unit 1.

The purpose of the "female" connector 16, is to allow the connection of the left electrode 12 and the right electrode 11, through the "male" connector 15, the left electrode wire 14 and of the right electrode wire 13 (check fig.2), to the electronic module 19, thus enabling the transmission of electrical impulses of low intensity and duration (of the "mini-electric shock" type), emitted by the latter, when the sensitivity level set by the user, at the moment of calibration, has been exceeded. The right 11 and left 12 electrodes are similar to those used in muscle electro-stimulation devices (TENS), with the side that comes into contact with the user's skin covered with a biocompatible, electroconductive adhesive film, manufactured of a flexible and easily compressible material. They are reusable for a large number of times. The purpose of this warning system, using low intensity and duration electrical impulses (of the "mini-electric shock" type), is to determine a higher response speed of the user, regarding the need to correct their posture at a certain moment, because the perceiving speed of an electrical stimulus is often faster than the perceiving speed of a sound stimulus. The latter can be easily disturbed by external factors. It also allows people with hearing impairments to be able to properly use the respective device.

The power supply of the electronic system 19 is ensured by a portable energy source, an alkaline or rechargeable battery, detachable and incorporated into said system 19.

The electronic system 19 has the following functions:
- to interpret the movements of the rod 20a, of the linear potentiometer 20 and to decide depending on the situation, the next step considering the purpose of the device, according to the invention;
- to allow the sensitivity level of the device to be set.

On the rear side of the housing 21, there is a longitudinal bossage 21a, which blocks the rotation of the elastic clip 17 (see fig.4). Due to this longitudinal bossage 21a and the screw 35, the elastic clip 17 can be mounted only in two positions, depending on the particularities of the strap / belt on which the central electronic unit 1 is located, namely: if a normal width strap/belt is used, the free ends are mounted at the bottom. If the used strap / belt is similar to the one used in strength training (abdominal protection belt), the free ends are mounted at the top, so as not to require large adjustments of the lengths of the cervical-thoracic thread, the left shoulder wire and the right shoulder thread, respectively, due to the large width of this type of belt / belt, in the lumbar area.

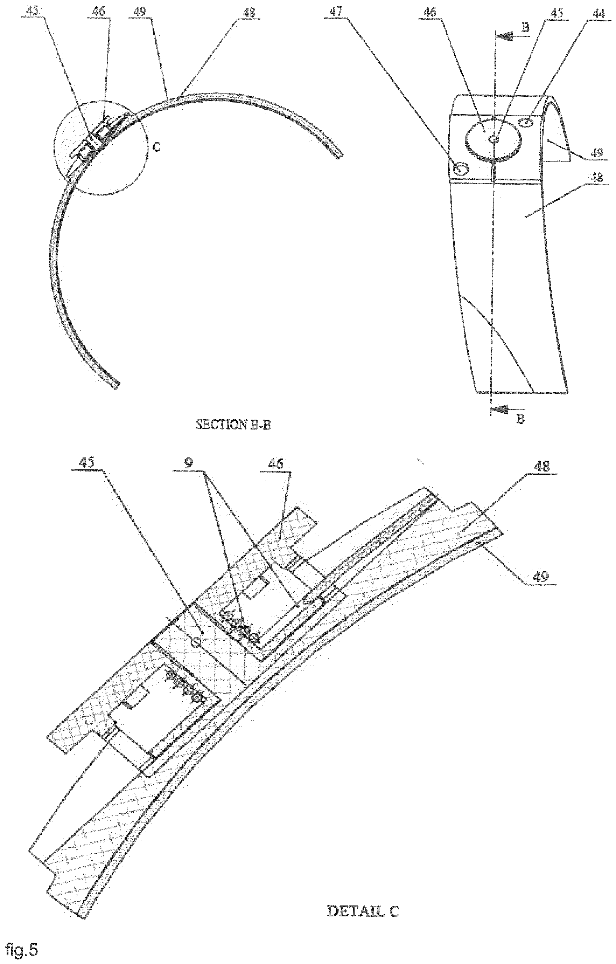

The three strap-type supports (right shoulder strap-type support 5, left shoulder strap-type support 8 and cervical strap-type support 6) are made identical from the constructive point of view. For example, take as a model the support of the left shoulder strap-type support 8 (see fig.5 and fig.6), which is composed of a belt 48, on the lower face of which is fixed a double-sided tape 49, an adjusting body 46, fixed rotatably on an anti-rotation body 45, embedded in the belt 48, on the upper face of the latter (see fig.5). On the adjusting body 46 of the left shoulder strap-type support 8, the left shoulder wire 9 is wound. On the adjusting body 37, of the right shoulder strap-type support 5, the right shoulder wire is wound 3. On the adjusting body 42, of the cervical strap-type support 6, the cervical-thoracic wire 4 is wound. All the 4 wires, the thoracic-lumbar wire 2, right shoulder wire 3, left shoulder wire 9 and cervical-thoracic wire 4, are made of a flexible and inextensible material. The belt 48 is made of a flexible material, preferably rubber with textile insertion to ensure breaking strength and the double-adhesive tape 49 has a biocompatible adhesive, which is reusable, flexible and easily compressible. If it wears out, it can be easily removed and replaced with another one of a same type, due to its doublesided adhesive. The adjusting body 46 has the shape of a spool. It has on the inner part of the upper end, a radially toothed area, 46a (see Fig. 6). The anti-rotation body 45, has the shape of a glass with a thick edge and presents an area, 45a, that has the conjugated shape of the toothed area 46a, of the adjusting body 46. At the center it has a stepped shaft, 45b, with two diameters. Its lower step is larger in diameter than the upper one. The adjusting body 46 has a central stepped bore 46b, with two diameters. Its lower step has a smaller diameter than the upper one, thus allowing the adjusting body 46, that is rotatably fixed on the central shaft 45b, to occupy two distinct positions, namely:
- when the adjusting body 46 is positioned on the antirotation body 45, having the lower step of the central bore 46b, placed on the upper step of the central axis 45b (check fig.6), it can rotate relatively easily due to the frictional clearance obtained between the parts in contact, thus allowing the winding of the left shoulder wire 9 on it, thus determining the shortening of the length of the respective wire and therefore its tension;
- when the adjusting body 46 is positioned on the antirotation body 45, having the lower stage of the central bore 46b, placed on the lower stage of the central axis 45b, it will rotate with great burden due to the tightening clearance obtained between the parts in contact. When the adjusting body 46 is completely pressed on the antirotation body 45, considering the tooths 46b enters the grooves 45a (check fig.5), the unwinding of the left shoulder wire 9 is completely blocked, thus maintaining its previously adjusted length.

In a similar way, the necessary lengths of the right shoulder wires 3 and the cervical-thoracic wire 4, can be adjusted using the adjusting body 37 and respectively the adjusting body 42. The free end of the central shaft, of each of the mentioned anti-rotation bodies, it is designed so that it can slide frictionally on the upper step of the central bore, of the corresponding adjusting body and lock during the transition from the upper to the lower step of the aforementioned bore, thus preventing the accidental removal of the adjusting body from the antirotation body on which it is fastened (check fig.6).

The thoracic-lumbar wire 2, presents a marking, that indicates the "0" position of the calibration. By positioning this marking, near the upper surface of the case 21, of the current device (see fig.3), the rod 20a, of the linear potentiometer 20, is brought, approximately, in the middle position of the latter's stroke. At this point, the electrical voltage of the output terminals, of the linear potentiometer 20, has its average value. This positioning is done by manipulating the thoracic-lumbar wire 2, until the aforementioned marking reaches the imposed position. Maintaining this previously obtained position is possible by screwing the retaining knob 18, until the end of its stroke, so that the free end of the shaft 18a, is pressed against the drive body 30, blocking its movement (check fig.4). The "0" position of the calibration represents the landmark depending on which the limits of the sensitivity levels of the device are established, according to the invention. Each of the three levels of sensitivity, of the device, in fact, represents a range of electric voltage values, generated by moving the rod 20a, of the linear potentiometer 20. The wider the range of values, the lower the level of sensitivity of the current device is and vice versa. The middle of each interval represents the average value of the electrical resistance, of the linear potentiometer 20 and therefore the average value of the electrical voltages generated at its output terminals. The limits of these intervals, of the device, according to the invention and therefore its sensitivity levels are preset by the software that manages the operation of the electronic system 19. The user cannot change those limits, but he can choose the desired sensitivity level, of the device in question, that he will use for a certain period of time.

The fastening support 7 comprises two rigid arms, left 50 and right 52, respectively, a locking knob 51, screwed on the connecting ends of the two arms mentioned above, a flexible body 55, that has its ends rigidly fixed on the outer ends of the two rigid arms, two pairs of magnets (56, 57) and (58, 59), one pair on each of the outer ends of the rigid arms, embedded on the lower faces of the two rigid arms and another pair of magnets (53, 54), embedded on the upper face of the flexible body 55, on either side of its symmetry plane (check fig.7 and fig.8). The magnet pairs (56, 57), (58, 59) and (53, 54) are partially embedded in the aforementioned elements, having a part of their body that protrudes outside those elements (check fig.7 and fig.8). The pairs of magnets embedded in the fastening support 7 have opposite polarities to the corresponding pairs embedded in the three strap-type supports mentioned above. The magnets embedded on the three strap-type supports are completely lowered inside some alveoli (check fig.5 and fig.10). Between the free face of each magnet and the upper face of each body, on which it is mounted, there is a gap with the purpose of allowing the free ends of the corresponding magnets, embedded on the fastening support 7, to penetrate inside it, preventing any lateral movement, so that during the established contact between the corresponding pairs of magnets, the strap-type supports remain fixed on the fastening support 7 (check. Fig. 1). The right rigid arm 52 has, at the free end, a recess which is meant to accomodate the free end of the left rigid arm 50, the latter being profiled accordingly. These two rigid arms are mounted rotatably, relative to each other, by means of the locking knob 51. Its threaded shaft passes through the through hole, made on the upper face of the free end of the right rigid arm 52, then through the through hole made on the free end of the left rigid arm 50 and it is then threaded into a corresponding hole, made on the lower side of the free end of the right rigid arm 52. The left rigid arm 50 and right rigid arm 52, exhibit on the common contact surfaces (50a, 52a and respectively 50b and 52b), radial grooves which have the role of preventing their rotation, one in relation to the other (see fig.9), if the locking knob 51 was rotated clockwise to the max and it is now in the locking position.

The fastening support 7 is placed on said three strap supports (right shoulder strap support 5, left shoulder strap support 8 and cervical strap support 6), maintaining the position thus obtained by means of the corresponding pairs of magnets on all four supports (check fig.1) namely: the pair of magnets (44, 47), embedded in the body of the support left shoulder bar 8 (check fig.10), corresponding to the pair of magnets (56, 57), embedded in the left rigid arm 50 of the fastening support 7 (check fig.8), the pair of magnets (36, 39), embedded in the body of the right shoulder bar support 5 (see fig. 10), corresponding to the pair of magnets (58, 59), embedded in the rigid arm 52 of the fastening support 7 and respectively the pair of magnets (41, 43), embedded in the body of the cervical bar support 6 (see fig.10), corresponding to the pair of magnets (53, 54), embedded in the flexible body 55 of the fastening support 7 (see fig. 7). The purpose of the fastening support 7 is to allow the user to place the three strap supports on their body, as easily as possible, without requiring the help of a third person, as well as to allow them to reposition the aforementioned elements, repeatedly, in the same positions, identical to the positions obtained at the time of calibrating the device, thus reducing the time required for subsequent calibrations (check fig.15).

There are two ways of placing the device, according to the invention, on the user's body, namely:
- placing the device without the help of a third party. This method requires the use of the fastening support 7;
- placing the device with the help of a third party.

Considering the device is ready to work, regardless of its placing method on the user's body, the adjusting bodies 37, 42 and 46 are positioned on the upper steps of the central shafts, of the anti-rotation bodies 38, 40 and 45 (check fig. 6), the right shoulder wire 3, cervical-thoracic wire 4 and the left shoulder wire 9, are completely unwound, so that their length is maximum. Thoracic-lumbar wire 2 is maneuvered until the "0" position of the calibration is reached, at which point the retaining knob 18 is screwed to the end of its stroke, thus determining the maintenance of the obtained position (check fig. 4).

The user removes the protective foils, which have been previously applied on the lower surfaces of the right shoulder strap-type support 5 and left shoulder strap-type support 8 and the cervical strap-type support 6. The purpose of these protective foils is to ensure the functional integrity of the adhesive layer of the aforementioned strap-type supports, while the respective device is not used; similar to those used to protect the electrodes of electro-stimulation devices (TENS).

If the user decides to place the device without the help of a third person, he places the fastening support 7 on the three supports, right shoulder strap-type support 5, left shoulder strap-type support 8 and cervical strap-type support 6. In this situation, the locking knob 51 is slightly loose so that the rigid arms, right 52 and left 50, can rotate, easily, in relation to each other.

The user turns on the central electronic unit 1, by pressing the ON / OFF button 25 and by repeatedly pressing the set button 24, he sets the desired sensitivity level of the device. For start, it is recommended to select a lower sensitivity level for the device, which can later be adjusted according to the user's needs. Once the desired sensitivity level is achieved, the device sends a visual confirmaton, by flashing the light on one of the low level indicator LED 26, medium level indicator LED 27, or high level indicator LED 28. The level setting is confirmed by emitting an uninterrupted sound signal for a few seconds, using the speaker 23 and by continuously flashing of the LED corresponding to the selection. The sensitivity level, thus obtained is maintained only if the user does not press the setting button 24 for more than three seconds. Changing the sensitivity level once the calibration is completed is possible only by continuously pressing for a few seconds the seting key 24. The reset action is confirmed by the device, through the speaker 23, which emits an audible uninterrupted signal, for five seconds.

The user inserts his head through the opening formed between the two rigid arms, left 50 and right 52 and the flexible body 55, of the fastening support 7, so that the central electronic unit 1 is resting on his back and he places on each shoulder the corresponding strap-type supports, so that the adjusting bodies 37 and 46 respectively are positioned approximately above the centers of rotation of the scapulo-humeral joints (check fig.15). The user then places the cervical strap-type support 6, so that the adjusting body 42 is positioned approximately above the *processes spinosus* of one of the cervical vertebrae C2 or C3 of the spine (approximately 2 cm from the base of the skull). In that area the hair does not significantly reduce the adhesion of the adhesive (present on the lower side of the cervical strap-type support 6) to the user's skin (check fig.15). Due to the adhesive present on the lower side of each strap-type support, the right shoulder strap-type support 5, cervical strap-type support 6 and left shoulder strap-type support 8, will be fixed without being able to detach or slide easily. It requires the action of an external force, significantly greater than the sum of the forces developed by the tensioning spring 31, of the central electronic unit 1, the frictional force between the drive body 30 and the guide body 33 and the frictional force between the rod 20a and the body of the linear potentiometer 20. After fixing the three strap-type supports, the user rotates the locking knob 51 clockwise, thus preventing the movement of the two rigid arms, left 50 and right 52, of the fastening support 7. Then, the user fixes and adjusts his abdominal belt 10, around the waist and moves the central electronic unit 1, which is placed on the abdominal belt 10, by means of the elastic clip 17, until the cutout 21a, through which the thoracic-lumbar wire 2 enters in the case 21, it will be positioned, approximately, next to the axis of symmetry of the user's spine. By turning the locking knob 51 clockwise, the angle between the right rigid arm 52 and the left rigid arm 50 is set (check fig.1). The positioning of the fastening support 7 on the two shoulder strap-type supports, mentioned above, is possible regardless of the distance between the user's shoulders, because the flexible body 55 is produced of a material with good flexibility. This feature facilitates its subsequent positioning / repositioning on the cervical strap-type support 6. Maintaining the angle between the two rigid arms, 50 and 52 allows the re-placement of the three strap-type supports, each time in the same position on the user's body, thus reducing the time required to recalibrate the respective device.

Then, the user removes the fastening support 7, detaching it from each of the three strap-type supports, one by one. The magnets that allowed its fastening on the three strap-type supports, are calculated in such a way that the pairs in contact cannot detach easily and the combined forces of attraction of the two pairs of magnets located on each of the three strap-type supports, do not exceed the adhesive force that maintains the contact between the lower surfaces of the three strap-type supports and the user's skin.

After removing the fastening support 7, the user proceeds to calibrate the device according to the invention. Thus, the user stands in a position as correct as possible, from the point of view of the spine and shoulders, taking into account his anatomical specificities, which he has to maintain throughout the calibration stage of the respective device and adjusts the tension on turns, on each of the three wires, right shoulder wire 3, left shoulder wire 9 and cervical-thoracic wire 4, by rotating clockwise the adjusting bodies 37, 46 and 42. Before placing the three strap-type supports on the user's body the three adjusting bodies will have to be loosened.

The tension in the three wires, right shoulder wire 3, left shoulder wire 9 and cervical-thoracic wire 4, implicitly produces a tension in the thoracic-lumbar wire 2. The first wire to have its tension adjusted is the cervical-thoracic wire 4. The adjustment of this wire is carried out using the hand with the best mobility / dexterity. In the shoulders case, it is necessary to operate the adjusting bodies with the help of the hand on the same side with the adjusting body because once the tension adjustment has been completed for the first shoulder wire, in order to be able to rotate the second adjusting body, the user can easily maintain the imposed posture, without moving the shoulders forward or twisting his spine. Otherwise, if the rotation is performed using the opposite hand to the adjusted parts, the shoulder of the hand that will rotate the second adjusting body, will move forward, thus further tensioning the already tensioned shoulder wire. This will lead to an improper calibration of the device according to the invention.

During the setting of the tension of the three wires mentioned above, the accidental rotation of the adjusting bodies is hampered as a result of the frictional clearance created between the lower step of the central bore of each adjusting body and the upper step of the central shaft of each corresponding anti-rotation body. Once the maximum number of rotations, that its necessary to acheive the right tension in each wire, has been achieved, on each of the three adjusting bodies, the latter are to be pressed completely down, so that the lower step of the central bore of each regulating body (37, 46 and 42 respectively), it is positioned on the lower step of the central shaft of the corresponding anti-rotation body (38, 45 and 40), respectively. The rotation of the adjusting bodies is thus completely blocked, due to the penetration of the teeths, present on the lower face of the adjusting bodies, in the grooves present on the upper face of the corresponding anti-rotation bodies (check fig.5). At this moment, all four wires, the right shoulder wire 3, the left shoulder wire 9, the cervical-thoracic wire 4 and thoracic-lumbar wire 2, are tensed, in contact and moulding with / on the user's back. If the user wishes a further, more precise adjustment of the tension in one or all of the previously mentioned wires, the user gently pulls up the adjusting body corresponding to the wire to be readjusted, until the teeth of its lower face disconnect from the grooves present on the upper side of the corresponding anti-rotation body and rotates, in one direction or another, the adjusting body, until he obtaines the desired tension in the wire. Once this fine adjustment has been completed, the user will press down on the previously operated adjusting body, thus completely blocking its rotation.

After completing the tension adjustments of the three wires, the right shoulder wire 3, the left shoulder wire 9, the cervical-thoracic wire 4 and implicitly the thoracic-lumbar wire 2, the user rotates the retaining knob 18 counterclockwise and releases the drive body 30, allowing it to move along with the thoracic-lumbar wire 2. At this moment, the calibration stage of the device, according to the invention, has ended and the actual utilisation stage begins.

The operating principle of the device, according to the invention, is the following: due to the inseparable connection between the four wires, the right shoulder wire 3, the left shoulder wire 9, the cervical-thoracic wire 4 and the thoracic-lumbar wire 2, their inextensibility, the fact that they have their free ends fixed, immovable (by means of the strap-type supports, right shoulder support 5, left shoulder support 8 and cervical support 6 and respectively of the drive body 30), on the right shoulder, on the left shoulder, at the level of the *processes spinosus* of one of the cervical vertebrae C2 or C3 (of the spine, of the user) and respectively on the rod 20a, of the linear potentiometer 20, as well as due to the pretensioning of these wires, accomplished in the calibration stage, corresponding to a posture that it's as close as possible (taking into account the anatomical specificities of the user, from the point of view of his spine and shoulders) to the physiological one, any subsequent chage in the curvature of the spine of the user or of his shoulders positions, will generate the change of the value of the electric voltage, at the output terminals of the linear potentiometer 20. The rod 20a, moves as a result of the traction applied on the thoracic-lumbar wire 2 (fixed on the upper end of the drive body 30- check fig.4), by one of the three wires, the right shoulder wire 3, the left shoulder wire 9 and / or the cervical-thoracic wire 4, in response to the user's postural change, compared to the posture obtained during the calibration stage of the device and as a result of the stretching force caused by the resort 31. Once the electronic system 19 of the central electronic unit 1, identifies the change in the electric voltage (in the output terminals of the linear potentiometer 20), compared to the one of the calibration phase of the device, it verifies if the voltage value is consistent with the sensitivity level of the device, set by the user. If the value is higher than the maximum value allowed according to the sensitivity level of the device, previously set by the user, then the electronic system 19 of the central electronic unit 1, will emit a continuous, audible warning signal, using the speaker 23 and will also emit a series of discontinuous electrical pulses, of low intensity and duration, noticeable on the user's skin, due to the contact between the right and left electrodes 12 and the user's body. The purpose of these warning signals is to cause the user to correct their posture, forcing them to return to the posture set during the calibration phase of the device. The signals in question cease once the user corrects their posture well enough so that the value of the electrical voltage, picked up at the output terminals of the linear potentiometer 20, is consistent with the level of sensitivity of the device, as previously set by the user. If the value of the voltage is in accordance with the sensitivity level of the device, as previously set by the user, the user may continue their activity unhindered.

The warning system using discontinuous electrical pulses, of low intensity and duration ("mini-electric shocks") is implicitly activated when the current device is turned on. However the "male" connector 15, is initially disconnected from the central electronic unit 1, while the "female" connector 16 is designed so it does not come into contact with the user's skin, to prevent any accidental electric shocks. If the user wishes to use this warning system with discontinuous electrical pulses, of low intensity and duration, applied via the two electrodes 11 and 12, he will first have to connect the "male" connector 15 to the "female" connector 16 and then place the electrodes 11 and 12, in a high muscle mass region (check fig.12 and fig.15), for example the buttock muscles (if the user especially wants to be warned about any improper postural changes of the lumbar spine), or the muscles covering the shoulder joints (if the user especially wants to be warned when improperly changing the position of the shoulders or the thoracic-cervical area of the spine). This warning system cannot be used by people wearing pacemaker implant, because the currents generated by the device according to the invention may interfere with the pacemaker's electrical signals.

The changes in the user's spine curvatures in the sagittal plane is noticed by the tensioning of the cervical-thoracic wire 4 and of the thoracic-lumbar wire 2 (check fig.11). The tensioning of the cervical-thoracic wire 4 produces the traction of the thoracic-lumbar wire 2. According to the operating principle of the device, according to the invention, stated above, the traction exerted on the thoraco-lumbar wire 2, due to changes in the user's spine curvatures, compared to the established posture set during the calibration stage (considered standard), determines the displacement of the rod 20a and therefore the modification of the electric voltage at the output terminals of the linear potentiometer 20. In this situation, the device will warn the user, emitting a sound signal and / or with a low intensity and low-duration electrical impulse, that their current position is incorrect, provided that the electronic module 19 of the central electronic unit 1, identifies that the electrical voltage recorded at the output terminals of the linear potentiometer 20, does not comply with the sensitivity level of the device, previously set by the user. For example, if the user wishes to bend forward to lift an object, so that it does not suffer a possible injury, the textbooks on the subject recommend that this movement is performed via the flexion at the hip joints. Thus, the purpose of the device will be to warn the user when they try to bend / straighten with a flexion / extension at the level of the spine (check fig.11 that shows the change in the position of the bifurcation point of the wires: right shoulder wire 3, left shoulder wire 9, cervical-thoracic wire 4 and thoraco-lumbar wire 2, which causes the change of the electric voltage value at the output terminals of the linear potentiometer 20 and can determine the emission of warning signals if the sensitivity level set by the user is exceeded).

The same principle applies in the case of the left and / or right shoulders forward movement. When the user moves one or both shoulders forward, meaning there is a change in the positions of the shoulders in relation to their position during calibration, the device identifies the change by tensioning the corresponding shoulder wire (right shoulder wire 3, left shoulder wire 9). As shown in the example of fig. 13, the forward movement of the right shoulder causes the displacement of the end of the right shoulder wire 3, that is anchored on the support of the right shoulder strap 5. Considering that the length of the wire cannot be changed (the wire is inextensible), the displacement of its end from the position it had in the calibration stage, causes the traction of the right shoulder wire 3 and therefore the displacement of the bifurcation point (Pbf) between the right shoulder wire 3 and the thoracic-lumbar wire 2. Due to the inseparable connection between the two wires and their inextensibility, the value of the electrical voltage changes at the output terminals of the linear potentiometer 20. The electronic system 19, of the central electronic unit 1, compares this newly obtained value, with the upper limit threshold of the sensitivity level, of the device, set prior during the calibration stage and treats it accordingly. The same principle applies to the forward movement of the left shoulder and / or of the simultaneous forward movement of both shoulders (check fig.12, fig.13 and fig.14).

After the user stops using the device, it is positioned so it does not cause warning signals and rotates the retaining knob 18 clockwise, thus blocking the drive body 30 and preventing its movement together with the movement of the thoracic-lumbar wire 2. Afterwards the user repositions the fastening support 7 on the right shoulder strap-type support 5, on the left shoulder strap-type support 8 and on the cervical strap-type support 6 and loosens the abdominal belt 10. Afterwards the user removes the fastening support 7 together with the entire device, detaching each of the three strap-type supports (right shoulder support 5, left shoulder support 8 and cervical support 6) from his body.

Considering the device it is ready to work, if the user chooses to place the device with the help of a third party, the fastening support 7 is no longer necessary. In this case, both the placement of the strap-type supports on the user's body and the adjustment of the tension in the right shoulder wire 3, cervical-thoracic wire 4 and left shoulder wire 9, will be performed by the third person, following the same procedure as described above. In this case, the adjustments imposed during the calibration stage of the device, according to the invention, are easier to perform and much more precise, since the user is not directly involved in their realization and is able to correctly maintain his imposed posture.

Regarding the secondary purpose of this invention, namely to allow the user to gradually obtain postural corrections, due to the possibility of setting the device's level of sensitivity, it enables the user to obtain postural corrections, lower or higher, depending on his anatomical needs and specificities. The device according to the invention, also allows the user to avoid being forced to adopt the physiological posture during the calibration stage (considered by the textbooks on the subject to be correct both from the point of view of the spine and shoulders), but allows them to adopt a posture as close as possible to the physiological one, taking into account its anatomical specificities. In this situation, the postural correction is performed step by step, starting from an incorrect initial posture and going through positions closer and closer to the physiological one. For example, in case of a user with certain deviations of the spine, such as kyphosis, or lordosis, he is not forced to adopt the physiological posture in the calibration stage of the respective device, however he will adopt, step by step, once a small or large correction is made, postures closer and closer to the correct one, until the final goal is reached. The physical and emotional effort to be made by the user are relatively small in this case, compared to those required by the physiotherapeutic exercises of postural correction imposed by the specialist. Also, through daily use, the device facilitates the achievement of the proposed goal in a relatively short time. Moreover, the device can be used in parallel with the execution of postural correction physiotherapeutic exercises, thus considerably reducing the time required to achieve the proposed goal.

With regard to the tertiary purpose of this invention, namely to allow the user to achieve and maintain a correct posture of the spine during walking, due to its size and light weight, the device can be used daily, regardless of the environment in which he operates. This device does not restrict the natural movements of the user's body, insofar as they are performed correctly from a biomechanical point of view, but warns him whenever the user's posture is not in accordance with the one imposed in the calibration stage.

Both the maintenance and the postural correction, performed with the help of the device, according to the invention, consist, in fact of autocorrections made by the user, as a reaction to the warning signals emitted by the respective device. Postural correction is obtained by cumulating several consecutive mini-corrections. These are due to the mental and physical adaptations of the user's body, to the positions imposed in each calibration stage corresponding to such a mini-correction session. Reaching the goal from a previous stage allows the imposition of maintaining a new position, closer to the physiological one, than the position from the previous stage.

The device, according to the invention, allows the user to opt for maintaining / correcting posture only from the point of view of his spine, or only from the point of view of his shoulders position. In order to achieve the first version of maintenance / postural correction, the user / third person has to adjust the tension of the cervical-thoracic wire 4, ignoring the adjustment of the tension of the left shoulder wire 9 and right shoulder wire 3. In the case of the second version of maintenance / postural correction, the user / third person should only adjust the tension of the left shoulder wire 9 and right shoulder wire 3, ignoring the adjustment of the cervical-thoracic wire 4.

## Claims

1. Device for real-time monitoring and active auto correction of a user's posture comprising:
- a central electronic unit (1) fastened on an adjustable abdominal belt (10);
- a flexible and inextensible thoracic-lumbar wire (2) connected to said central electronic unit (1), said thoracic-lumbar wire (2) having a bifurcation point (Pbf) corresponding to a proximal end of:
- a right shoulder wire (3),
- a left shoulder wire (9) and
- a cervical-thoracic wire (4) and each of said right shoulder, left shoulder and cervical-thoracic wires (3, 9, 4) being inextensible and having a distal end, opposite said bifurcation point (Pbf), fixed on a corresponding right shoulder strap-type support (5), on a left shoulder strap-type support (8) and on a cervical strap-type support (6),
said central electronic unit (1) comprising:
- a case (21) defining an interior volume wherein a circular opening (21a) is provided in an upper wall of the case (21) through which said flexible and inextensible thoracic-lumbar wire (2) is disposed inside the central electronic unit (1),
said thoracic-lumbar wire (2) being further connected to an upper end of a drive body (30) and
- signaling means (22, 23, 26, 27, 28) connected to an electronic module (19) provided with a circuit board which is further connected to a portable energy source
**characterized in that**
each of said strap-type supports (5, 8, 6) has a reusable double-sided biocompatible adhesive tape (49) for adhesion to an user's body and said right shoulder strap-type support (5) and said left shoulder strap-type support (8) are configured to secure said right shoulder wire (3) and said left shoulder wire (9) on a right and respectively left shoulder joint area of the user via said adhesive tape (49), and said cervical strap-type support (6) is configured to secure said cervical-thoracic wire (4) on a cervical area of the user via said adhesive tape (49),
**and in that** said electronic module (19) is further connected to a linear potentiometer (20) with output terminals, said potentiometer (20) having a rod (20a) and said drive body (30), fixed to the rod (20a), wherein the drive body (30) is slidingly mounted inside a guide body (33), fixed on the circuit board of the electronic module (19);
- a lower end of said drive body (30) is connected to a tension spring (31) and further to a rotating hook (32) which is placed between the tension spring (31) and the guide body (33),
**such that** upon traction or looseness exerted on the thoracic-lumbar wire (2), by any of the right shoulder wire (3), left shoulder wire (9) and/or cervical-thoracic wire (4), caused by changes in the user's posture with regards to a pre-established calibrated normal posture, marked on the thoracic-lumbar wire (2), the drive body (30) causes the rod (20a) of the linear potentiometer (20) to slide along the guide body (33) in a bi-directional manner due to said tension spring (31) and to produce a modified value of an electrical voltage at the output terminals of said linear potentiometer (20) triggering the electronic module (19) to compare said modified value with a lower limit or an upper limit value of a range of values of electrical voltages corresponding to a level of sensibility of the device pre-set by the user and to send a warning signal of an abnormal posture perceptible by the user when said modified value exceeds said lower or upper limit value.

2. Device for real-time monitoring and active auto correction of a user's posture according to claim 1 wherein each of said strap-type supports (5, 8, 6) comprises:
- a strap (48) made of a flexible material, preferably rubber with textile insertion having said adhesive tape (49) placed on its lower surface, facing the shoulder joint area or the cervical area of a user, and having on said strap's (48) upper surface, a body (38, 40, 45) which is partially embedded on said upper surface and an adjusting body (46, 37, 42), rotatably mounted on said body (38, 40, 45).

3. Device for real-time monitoring and active auto correction of a user's posture according to claim 2 wherein each of said strap-type supports (5, 8, 6) further comprises a first pair of magnets (36, 39, 44, 47, 41, 43) embedded on said strap's (48) upper surface inside corresponding alveoli and having a gap between each magnet (36, 39, 44, 47, 41, 43) and said strap's (48) upper surface.

4. Device for real-time monitoring and active auto correction of a user's posture according to claim 2 or 3 wherein said adjusting body (46, 37, 42) has a spool shape.

5. Device for real-time monitoring and active auto correction of a user's posture according to claims 2 - 4 wherein said right shoulder wire (3) is wound on the adjusting body (37) of the right shoulder strap-type support (5), said left shoulder wire (9) is wound on the adjusting body (46) of the left shoulder strap-type support (8) and said cervical-thoracic wire (4) is wound on the adjusting body (42) of the cervical strap-type support (6).

6. Device for real-time monitoring and active auto correction of a user's posture according to claims 2 - 5 wherein
said adjusting body (46, 37, 42) comprises:
- a radially toothed area (46a) provided on an inner part of an upper end of said adjusting body (46, 37, 42) and
- a central stepped bore (46b) with a lower step and an upper step, said lower step having a smaller diameter compared to a diameter of its upper step and
said body (38, 40, 45) comprises:
- an area (45a) having a conjugated shape for coupling with the radially toothed area (46a) of said adjusting body (46, 37, 42) and
- a central stepped shaft (45b) with a lower step and an upper step,
said lower step having a larger diameter compared to a diameter of its upper step
wherein said adjusting body (46, 37, 42) is rotatably mounted on said central stepped shaft (45b) of said body (38, 40, 45).

7. Device for real-time monitoring and active auto correction of a user's posture according to any of the preceding claims wherein said electronic module (19) of the central electronic unit (1) is connected to two TENS-type electrodes (11, 12) placed at an outer end of a right electrode wire (13) and respectively at an outer end of a left electrode wire (14).

8. Device for real-time monitoring and active auto correction of a user's posture according to claim 7 wherein said TENS-type electrodes (11, 12) have on a side that comes into contact with the user's skin, a biocompatible, electro conductive adhesive film, made of a flexible, reusable and easily compressible material.

9. Device for real-time monitoring and active auto correction of a user's posture according to any of the preceding claims wherein said portable energy source of the electronic module (19) is preferably an alkaline or rechargeable battery.

10. Device for real-time monitoring and active auto correction of a user's posture according to any of the preceding claims wherein the central electronic unit (1) is fastened on the adjustable abdominal belt (10) by means of an elastic clip (17) fixed by a screw (35) on said case (21).

11. Device for real-time monitoring and active auto correction of a user's posture according to any of the preceding claims wherein said case (21) further comprises a retaining knob (18) which is arranged to penetrate through a recess in a side wall of the guide body (33), such that, when in use, a free end (18a) of the retaining knob (18) is arranged to reach in a vicinity of the drive body (30) to block the sliding movement of the drive body (30) and of the rod (20a) to allow the calibration of the device.

12. Device for real-time monitoring and active auto correction of a user's posture according to claims 3-11, further enclosing a fastening support (7) which comprises:
- a left rigid arm (50) and a right rigid arm (52), each said arms (50, 52) having a connecting end and an outer end; each of said connecting ends having a through hole which extends transversally from a front side of the rigid arms (50, 52) to a back side of the rigid arms (50, 52) and
said left rigid arm (50) comprises a profiled protrusion provided at its connecting end and said right rigid arm (52) comprises a corresponding profiled recess provided at its connecting end which is arranged to receive the corresponding profiled protrusion of said left rigid arm (50);
- a flexible body (55) with two ends, each said end being rigidly fixed on an outer end of the two rigid arms (50, 52) to form an open enclosure;
- a locking knob (51) screwed on the connecting ends of the two rigid arms (50, 52) such that said two rigid arms (50, 52) are rotatably mounted relative to each other, in a vertical plane;
- two second pairs of magnets (56, 57, 58, 59), each second pair of magnets (56, 57, 58, 59) being partially embedded on a lower face of each outer end of the two rigid arms (50, 52);
- a third pair of magnets (53, 54) partially embedded on an upper face of said flexible body (55) on either side of its vertical symmetry plane and
said two second pairs of magnets (56, 57, 58, 59) and said third pair of magnets (53, 54) having opposite polarities to their corresponding first pair of magnets (36, 39, 44, 47, 41, 43) embedded on said strap's (48) upper surface and placed **such that** upon contact between the corresponding pairs of magnets, the strap-type supports (5, 8, 6) remain fixed on the fastening support (7).

13. Device for real-time monitoring and active auto correction of a user's posture according to claim 12 wherein the left rigid arm (50) and the right rigid arm (52) have a plurality of common contact surfaces (50a, 52a, 50b, 52b) which comprise radial grooves.

## Patentansprüche

1. Vorrichtung zur Echtzeitüberwachung und aktiven automatischen Korrektur der Körperhaltung eines Benutzers, umfassend:
- eine zentrale elektronische Einheit (1), die an einem verstellbaren Bauchgurt (10) befestigt ist;
- einen flexiblen und nicht dehnbaren thorakal-lumbalen Draht (2), der mit der zentralen elektronischen Einheit (1) verbunden ist, wobei der thorakal-lumbale Draht (2) einen Verzweigungspunkt (Pbf) aufweist, der einem proximalen Ende:
- eines rechten Schulterdrahtes (3),
- eines linken Schulterdrahtes (9) und
- eines zervikal-thorakalen Drahtes (4) entspricht
und jeder der rechten Schulter-, linken Schulter- und zervikal-thorakalen Drähte (3, 9, 4) nicht dehnbar ist und ein distales Ende aufweist, das dem Verzweigungspunkt (Pbf) gegenüberliegt und an einer entsprechenden gurtartigen Halterung für die rechte Schulter (5), an einer gurtartigen Halterung für die linke Schulter (8) und an einer zervikalen gurtartigen Halterung (6) ortsfest angebracht ist,
wobei die zentrale elektronische Einheit (1) Folgendes umfasst:
- ein Gehäuse (21), das ein Innenvolumen definiert, wobei eine kreisförmige Öffnung (21a) in einer oberen Wand des Gehäuses (21) vorgesehen ist, durch die der flexible und nicht dehnbare thorakal-lumbale Draht (2) innerhalb der zentralen elektronischen Einheit (1) angeordnet ist,
wobei der thorakal-lumbale Draht (2) ferner mit einem oberen Ende eines Antriebskörpers (30) verbunden ist und
- Signalisierungsmittel (22, 23, 26, 27, 28), die mit einem elektronischen Modul (19) verbunden sind, das mit einer Leiterplatte versehen ist, die wiederum mit einer tragbaren Energiequelle verbunden ist
**dadurch gekennzeichnet, dass**
jede der gurtartigen Halterungen (5, 8, 6) ein wiederverwendbares doppelseitiges biokompatibles Klebeband (49) zum Ankleben an den Körper eines Benutzers aufweist und die gurtartige Halterung für die rechte Schulter (5) und die gurtartige Halterung für die linke Schulter (8) derart ausgebildet sind, dass sie den rechten Schulterdraht (3) und den linken Schulterdraht (9) an einem rechten bzw. linken Schultergelenkbereich des Benutzers über das Klebeband (49) befestigen, und die zervikale gurtartige Halterung (6) derart ausgebildet ist, dass sie den zervikal-thorakalen Draht (4) an einem Zervikalbereich des Benutzers über das Klebeband (49) befestigt,
**und dass** das elektronische Modul (19) ferner mit einem linearen Potentiometer (20) mit Ausgangsanschlüssen verbunden ist, wobei das Potentiometer (20) eine Stange (20a) und den Antriebskörper (30) aufweist, der an der Stange (20a) befestigt ist, wobei der Antriebskörper (30) gleitend innerhalb eines Führungskörpers (33) angebracht ist, der auf der Leiterplatte des elektronischen Moduls (19) befestigt ist;
- ein unteres Ende des Antriebskörpers (30) mit einer Zugfeder (31) und ferner mit einem drehbaren Haken (32) verbunden ist, der zwischen der Zugfeder (31) und dem Führungskörper (33) angeordnet ist,
**so dass** bei Zug oder Lockerheit, die auf den thorakal-lumbalen Draht (2) durch einen der rechten Schulterdraht (3), linken Schulterdraht (9) und/oder zervikal-thorakalen Draht (4) ausgeübt wird, die durch Änderungen der Körperhaltung des Benutzers in Bezug auf eine vorbestimmte kalibrierte normale Körperhaltung verursacht werden, die auf dem thorakal-lumbalen Draht (2) markiert ist, der Antriebskörper (30) die Stange (20a) des linearen Potentiometers (20) veranlasst, aufgrund der Zugfeder (31) in zwei Richtungen entlang des Führungskörpers (33) zu gleiten und einen modifizierten Wert einer elektrischen Spannung an den Ausgangsanschlüssen des linearen Potentiometers (20) zu erzeugen, wodurch das elektronische Modul (19) veranlasst wird, den modifizierten Wert mit einem unteren Grenzwert oder einem oberen Grenzwert eines Bereichs von Werten elektrischer Spannungen zu vergleichen, die einem vom Benutzer voreingestellten Empfindlichkeitsgrad der Vorrichtung entsprechen, und ein Warnsignal für eine vom Benutzer wahrnehmbare abnormale Körperhaltung zu senden, wenn der veränderte Wert den unteren oder oberen Grenzwert überschreitet.

2. Vorrichtung zur Echtzeitüberwachung und aktiven automatischen Korrektur der Körperhaltung eines Benutzers nach Anspruch 1, wobei jede der gurtartigen Halterungen (5, 8, 6) Folgendes umfasst:
- einen Gurt (48) aus einem flexiblen Material, vorzugsweise Gummi mit einer Textileinlage, der auf seiner Unterseite das Klebeband (49) aufweist, wobei der Gurt dem Schultergelenkbereich oder dem Zervikalbereich eines Benutzers zugewandt ist und auf der Oberseite des Gurtes (48) einen teilweise in die Oberseite eingebetteten Körper (38, 40, 45) und einen drehbar an dem Körper (38, 40, 45) angebrachten Einstellkörper (46, 37, 42) aufweist.

3. Vorrichtung zur Echtzeitüberwachung und aktiven automatischen Korrektur der Körperhaltung eines Benutzers nach Anspruch 2, wobei jede der gurtartigen Halterungen (5, 8, 6) ferner ein erstes Paar von Magneten (36, 39, 44, 47, 41, 43) umfasst, das auf der Oberseite des Gurtes (48) innerhalb entsprechender Hohlräume eingebettet ist und einen Spalt zwischen jedem Magneten (36, 39, 44, 47, 41, 43) und der Oberseite des Gurtes (48) aufweist.

4. Vorrichtung zur Echtzeitüberwachung und aktiven automatischen Korrektur der Körperhaltung eines Benutzers nach Anspruch 2 oder 3, wobei der Einstellkörper (46, 37, 42) eine Spulenform aufweist.

5. Vorrichtung zur Echtzeitüberwachung und aktiven automatischen Korrektur der Körperhaltung eines Benutzers nach einem der Ansprüche 2 bis 4, wobei der rechte Schulterdraht (3) auf den Einstellkörper (37) der gurtartigen Halterung für die rechte Schulter (5) gewickelt ist, der linke Schulterdraht (9) auf den Einstellkörper (46) der gurtartigen Halterung für die linke Schulter (8) gewickelt ist und der zervikalthorakale Draht (4) auf den Einstellkörper (42) der zervikalen gurtartigen Halterung (6) gewickelt ist.

6. Vorrichtung zur Echtzeitüberwachung und aktiven automatischen Korrektur der Körperhaltung eines Benutzers nach einem der Ansprüche 2 bis 5, wobei
der Einstellkörper (46, 37, 42) Folgendes umfasst:
- einen radial gezahnten Bereich (46a), der an einem inneren Teil eines oberen Endes des Einstellkörpers (46, 37, 42) vorgesehen ist, und
- eine zentrale Stufenbohrung (46b) mit einer unteren Stufe und einer oberen Stufe, wobei die untere Stufe einen kleineren Durchmesser im Vergleich zu einem Durchmesser der oberen Stufe aufweist, und
der Körper (38, 40, 45) Folgendes umfasst:
- einen Bereich (45a) mit einer abgestimmten Form zur Kopplung mit dem radial gezahnten Bereich (46a) des Einstellkörpers (46, 37, 42) und
- eine zentrale Stufenwelle (45b) mit einer unteren Stufe und einer oberen Stufe, wobei die untere Stufe einen größeren Durchmesser im Vergleich zu einem Durchmesser der oberen Stufe aufweist
wobei der Einstellkörper (46, 37, 42) drehbar auf der zentralen Stufenwelle (45b) des Körpers (38, 40, 45) angebracht ist.

7. Vorrichtung zur Echtzeitüberwachung und aktiven automatischen Korrektur der Körperhaltung eines Benutzers nach einem der vorhergehenden Ansprüche, wobei das elektronische Modul (19) der zentralen elektronischen Einheit (1) mit zwei TENS-Elektroden (11, 12) verbunden ist, die an einem äußeren Ende eines rechten Elektrodendrahtes (13) bzw. an einem äußeren Ende eines linken Elektrodendrahtes (14) angeordnet sind.

8. Vorrichtung zur Echtzeitüberwachung und aktiven automatischen Korrektur der Körperhaltung eines Benutzers nach Anspruch 7, wobei die TENS-Elektroden (11, 12) auf einer mit der Haut des Benutzers in Kontakt kommenden Seite eine biokompatible, elektrisch leitende Klebefolie aus einem flexiblen, wiederverwendbaren und leicht komprimierbaren Material aufweisen.

9. Vorrichtung zur Echtzeitüberwachung und aktiven automatischen Korrektur der Körperhaltung eines Benutzers nach einem der vorhergehenden Ansprüche, wobei die tragbare Energiequelle des elektronischen Moduls (19) vorzugsweise eine alkalische oder wiederaufladbare Batterie ist.

10. Vorrichtung zur Echtzeitüberwachung und aktiven automatischen Korrektur der Körperhaltung eines Benutzers nach einem der vorhergehenden Ansprüche, wobei die zentrale elektronische Einheit (1) an dem verstellbaren Bauchgurt (10) mit Hilfe eines mittels einer Schraube (35) an dem Gehäuse (21) befestigten elastischen Clips (17) angebracht ist.

11. Vorrichtung zur Echtzeitüberwachung und aktiven automatischen Korrektur der Körperhaltung eines Benutzers nach einem der vorhergehenden Ansprüche, wobei das Gehäuse (21) ferner einen Rückhalteknopf (18) umfasst, der so angeordnet ist, dass er durch eine Aussparung in einer Seitenwand des Führungskörpers (33) eindringt, so dass beim Gebrauch ein freies Ende (18a) des Rückhalteknopfes (18) so angeordnet ist, dass es in die Nähe des Antriebskörpers (30) reicht, um die Gleitbewegung des Antriebskörpers (30) und der Stange (20a) zu blockieren und die Kalibrierung der Vorrichtung zu ermöglichen.

12. Vorrichtung zur Echtzeitüberwachung und aktiven automatischen Korrektur der Körperhaltung eines Benutzers nach den Ansprüchen 3-11, die ferner einen Befestigungsträger (7) aufweist, der Folgendes umfasst:
- einen linken starren Arm (50) und einen rechten starren Arm (52), wobei jeder der Arme (50, 52) ein Verbindungsende und ein äußeres Ende aufweist; wobei jedes der Verbindungsenden ein Durchgangsloch aufweist, das sich quer von einer Vorderseite der starren Arme (50, 52) zu einer Rückseite der starren Arme (50, 52) erstreckt, und
der linke starre Arm (50) einen an dessen Verbindungsende vorgesehenen profilierten Vorsprung aufweist und der rechte starre Arm (52) eine an dessen Verbindungsende vorgesehene entsprechende profilierte Aussparung aufweist, die so angeordnet ist, dass sie den entsprechenden profilierten Vorsprung des linken starren Arms (50) aufnimmt;
- einen biegsamen Körper (55) mit zwei Enden, wobei jedes dieser Enden ortsfest an einem äußeren Ende der beiden starren Arme (50, 52) befestigt ist, um eine offene Umhüllung zu bilden;
- einen Verriegelungsknopf (51), der auf die Verbindungsenden der beiden starren Arme (50, 52) geschraubt ist, so dass die beiden starren Arme (50, 52) relativ zueinander in einer vertikalen Ebene drehbar angebracht sind;
- zwei zweite Paare von Magneten (56, 57, 58, 59), wobei jedes zweite Paar von Magneten (56, 57, 58, 59) an einer unteren Fläche jedes äußeren Endes der beiden starren Arme (50, 52) teilweise eingebettet ist;
- ein drittes Paar von Magneten (53, 54), das teilweise an einer oberen Fläche des flexiblen Körpers (55) auf beiden Seiten dessen vertikalen Symmetrieebene eingebettet ist und
wobei die beiden zweiten Paare von Magneten (56, 57, 58, 59) und das dritte Paar von Magneten (53, 54) entgegengesetzte Polaritäten zu ihrem entsprechenden ersten Paar von Magneten (36, 39, 44, 47, 41, 43) aufweisen, das auf der Oberseite des Gurtes (48) eingebettet und **so** angeordnet ist, **dass** bei Kontakt zwischen den entsprechenden Paaren von Magneten die gurtartigen Halterungen (5, 8, 6) ortsfest auf dem Befestigungsträger (7) bleiben.

13. Vorrichtung zur Echtzeitüberwachung und aktiven automatischen Korrektur der Körperhaltung eines Benutzers nach Anspruch 12, wobei der linke starre Arm (50) und der rechte starre Arm (52) eine Vielzahl gemeinsamer Kontaktflächen (50a, 52a, 50b, 52b) aufweisen, die radialen Nuten umfassen.

## Revendications

1. Dispositif de surveillance en temps réel et d'autocorrection active de la posture d'un utilisateur comprenant :
- une unité électronique centrale (1) fixée sur une ceinture abdominale réglable (10);
- un fil thoraco-lombaire flexible et inextensible (2) relié à ladite unité électronique centrale (1), ledit fil thoraco-lombaire (2) ayant un point de bifurcation (Pbf) correspondant à une extrémité proximale:
- d'un fil d'épaule droit (3),
- d'un fil d'épaule gauche (9) et
- d'un fil cervico-thoracique (4)
et chacun desdits fils d'épaule droite, d'épaule gauche et cervico-thoracique (3, 9, 4) étant inextensible et ayant une extrémité distale, opposée audit point de bifurcation (Pbf), fixée sur un support correspondant de type sangle d'épaule droit (5), sur un support de type sangle d'épaule gauche (8) et sur un support de type sangle cervicale (6),
ladite unité électronique centrale (1) comprenant:
- un boîtier (21) définissant un volume intérieur dans lequel une ouverture circulaire (21a) est prévue dans une paroi supérieure du boîtier (21) à travers laquelle ledit fil thoraco-lombaire flexible et inextensible (2) est disposé à l'intérieur de l'unité électronique centrale (1),
ledit fil thoraco-lombaire (2) étant en outre relié à une extrémité supérieure d'un corps d'entraînement (30), et
- des moyens de signalisation (22, 23, 26, 27, 28) reliés à un module électronique (19) doté d'une carte de circuit imprimé qui est en outre relié à une source d'énergie portable
**caractérisé en ce que**
chacun desdits supports de type sangle (5, 8, 6) possède une bande adhésive biocompatible double face réutilisable (49) pour adhérer au corps de l'utilisateur et ledit support de type sangle d'épaule droit (5) et ledit support de type sangle d'épaule gauche (8) sont configurés pour fixer ledit fil d'épaule droit (3) et ledit fil d'épaule gauche (9) sur une zone d'articulation d'épaule droit et respectivement gauche de l'utilisateur par l'intermédiaire de ladite bande adhésive (49), et ledit support de type sangle cervicale (6) est configuré pour fixer ledit fil cervico-thoracique (4) sur une zone cervicale de l'utilisateur par l'intermédiaire dudit bande adhésif (49),
**et en ce que** ledit module électronique (19) est en outre connecté à un potentiomètre linéaire (20) avec des bornes de sortie, ledit potentiomètre (20) ayant une tige (20a) et ledit corps d'entraînement (30), fixé à la tige (20a), dans lequel le corps d'entraînement (30) est monté coulissant à l'intérieur d'un corps de guidage (33), fixé sur la carte de circuit imprimé du module électronique (19);
- une extrémité inférieure dudit corps d'entraînement (30) est reliée à un ressort de tension (31) et en outre à un crochet rotatif (32) qui est placé entre le ressort de tension (31) et le corps de guidage (33),
**de telle sorte** qu' en cas de traction ou de relâchement exercé sur le fil thoraco-lombaire (2) par l'un quelconque des fils d'épaule droit (3), d'épaule gauche (9) et/ou du fil cervico-thoracique (4), causé par des changements dans la posture de l'utilisateur par rapport à une posture normale calibrée préétablie, marquée sur le fil thoraco-lombaire (2), le corps d'entraînement (30) fait glisser la tige (20a) du potentiomètre linéaire (20) le long du corps de guidage (33) de manière bidirectionnelle grâce audit ressort de traction (31) et produit une valeur modifiée d'une tension électrique aux bornes de sortie dudit potentiomètre linéaire (20) déclenchant le module électronique (19) pour comparer ladite valeur modifiée avec une valeur limite inférieure ou limite supérieure d'une plage de valeurs de tensions électriques correspondant à un niveau de sensibilité du dispositif préétabli par l'utilisateur et pour envoyer un signal d'avertissement d'une posture anormale perceptible par l'utilisateur lorsque ladite valeur modifiée dépasse ladite valeur limite inférieure ou supérieure.

2. Dispositif de surveillance en temps réel et d'autocorrection active de la posture d'un utilisateur selon la revendication 1, dans lequel chacun des supports de type sangle (5, 8, 6) comprend:
- une sangle (48) faite d'un matériau flexible, de préférence du caoutchouc avec une insertion textile ayant ladite bande adhésive (49) placée sur sa surface inférieure, orientée vers la zone de l'articulation de l'épaule ou la zone cervicale d'un utilisateur, et ayant sur la surface supérieure de ladite sangle (48), un corps (38, 40, 45) qui est partiellement encastré sur ladite surface supérieure et un corps de réglage (46, 37, 42), monté de manière rotative sur ledit corps (38, 40, 45).

3. Dispositif de surveillance en temps réel et d'autocorrection active de la posture d'un utilisateur selon la revendication 2, dans lequel chacun des supports de type sangle (5, 8, 6) comprend en outre une première paire d'aimants (36, 39, 44, 47, 41, 43) encastrés sur la surface supérieure de ladite sangle (48) à l'intérieur d'alvéoles correspondantes et présentant un espace entre chaque aimant (36, 39, 44, 47, 41, 43) et la surface supérieure de ladite sangle (48).

4. Dispositif de surveillance en temps réel et d'autocorrection active de la posture d'un utilisateur selon la revendication 2 ou 3, dans lequel ledit corps de réglage (46, 37, 42) a une forme de bobine.

5. Dispositif de surveillance en temps réel et d'autocorrection active de la posture d'un utilisateur selon les revendications 2 à 4, dans lequel le fil d'épaule droit (3) est enroulé sur le corps de réglage (37) du support de type sangle d'épaule droit (5), ledit fil d'épaule gauche (9) est enroulé sur le corps de réglage (46) du support de type sangle d'épaule gauche (8) et ledit fil cervico-thoracique (4) est enroulé sur le corps de réglage (42) du support de type sangle cervicale (6).

6. Dispositif de surveillance en temps réel et d'autocorrection active de la posture d'un utilisateur selon les revendications 2 à 5, dans lequel
ledit corps de réglage (46, 37, 42) comprend:
- une zone radialement dentée (46a) située sur une partie intérieure de l'extrémité supérieure dudit corps de réglage (46, 37, 42) et
- un alésage central à gradins (46b) avec un gradin inférieur et un gradin supérieur, ledit gradin inférieur ayant un diamètre inférieur au diamètre de son gradin supérieur et
ledit corps (38, 40, 45) comprend:
- une zone (45a) ayant une forme conjuguée pour s'accoupler avec la zone radialement dentée (46a) dudit corps de réglage (46, 37, 42) et
- un arbre central à gradins (45b) avec un gradin inférieur et un gradin supérieur, ledit gradin inférieur ayant un diamètre plus grand que le diamètre de son gradin supérieur
dans lequel ledit corps de réglage (46, 37, 42) est monté rotatif sur ledit arbre central à gradins (45b) dudit corps (38, 40, 45).

7. Dispositif de surveillance en temps réel et d'autocorrection active de la posture d'un utilisateur selon l'une quelconque des revendications précédentes, dans lequel ledit module électronique (19) de l'unité électronique centrale (1) est connecté à deux électrodes de type TENS (11, 12) placées à une extrémité extérieure d'un fil d'électrode droit (13) et respectivement à une extrémité extérieure d'un fil d'électrode gauche (14).

8. Dispositif de surveillance en temps réel et d'autocorrection active de la posture d'un utilisateur selon la revendication 7, dans lequel lesdites électrodes de type TENS (11, 12) présentent, sur une face en contact avec la peau de l'utilisateur, un film adhésif biocompatible et électro conducteur, constitué d'un matériau souple, réutilisable et facilement compressible.

9. Dispositif de surveillance en temps réel et d'autocorrection active de la posture d'un utilisateur selon l'une quelconque des revendications précédentes, dans lequel ladite source d'énergie portable du module électronique (19) est de préférence une pile alcaline ou rechargeable.

10. Dispositif de surveillance en temps réel et d'autocorrection active de la posture d'un utilisateur selon l'une quelconque des revendications précédentes, dans lequel l'unité électronique centrale (1) est fixée sur la ceinture abdominale réglable (10) au moyen d'un clip élastique (17) fixé par une vis (35) sur ledit boîtier (21).

11. Dispositif de surveillance en temps réel et d'autocorrection active de la posture d'un utilisateur selon l'une quelconque des revendications précédentes, dans lequel ledit boîtier (21) comprend en outre un bouton de retenue (18) qui est disposé pour pénétrer à travers un renfoncement dans une paroi latérale du corps de guidage (33), de sorte que, lors de l'utilisation, une extrémité libre (18a) du bouton de retenue (18) est disposée pour atteindre la proximité du corps d'entraînement (30) afin de bloquer le mouvement de glissement du corps d'entraînement (30) et de la tige (20a) afin de permettre la calibration de l'appareil.

12. Dispositif de surveillance en temps réel et d'autocorrection active de la posture d'un utilisateur selon les revendications 3 à 11, renfermant en outre un support de fixation (7) qui comprend:
- un bras rigide gauche (50) et un bras rigide droit (52), chacun desdits bras (50, 52) ayant une extrémité de connexion et une extrémité extérieure; chacune desdites extrémités de connexion ayant un trou traversant qui s'étend transversalement d'un côté avant des bras rigides (50, 52) à un côté arrière des bras rigides (50, 52) et
ledit bras rigide gauche (50) comprend une protubérance profilée prévue à son extrémité de connexion et ledit bras rigide droit (52) comprend un renfoncement profilé correspondant prévu à son extrémité de connexion qui est disposé pour recevoir la protubérance profilée correspondante dudit bras rigide gauche (50);
- un corps flexible (55) à deux extrémités, chacune desdites extrémités étant fixée rigidement sur une extrémité extérieure des deux bras rigides (50, 52) pour former une enceinte ouverte;
- un bouton de verrouillage (51) vissé sur les extrémités de connexion des deux bras rigides (50, 52) de sorte que lesdits deux bras rigides (50, 52) sont montés rotatifs l'un par rapport à l'autre, dans un plan vertical;
- deux deuxièmes paires d'aimants (56, 57, 58, 59), chaque deuxième paire d'aimants (56, 57, 58, 59) étant partiellement encastrée sur une face inférieure de chaque extrémité extérieure des deux bras rigides (50, 52);
- une troisième paire d'aimants (53, 54) partiellement encastrée sur une face supérieure dudit corps flexible (55) de part et d'autre de son plan de symétrie vertical et
lesdites deux deuxièmes paires d'aimants (56, 57, 58, 59) et ladite troisième paire d'aimants (53, 54) ayant des polarités opposées à leurs premières paires d'aimants correspondantes (36, 39, 44, 47, 41, 43) encastrées sur la surface supérieure de ladite sangle (48) et placées de **telle sorte qu**'au contact entre les paires d'aimants correspondantes, les supports de type sangle (5, 8, 6) restent fixés sur le support de fixation (7).

13. Dispositif de surveillance en temps réel et d'autocorrection active de la posture d'un utilisateur selon la revendication 12, dans lequel le bras rigide gauche (50) et le bras rigide droit (52) ont une pluralité de surfaces de contact communes (50a, 52a, 50b, 52b) qui comprennent des rainures radiales.
